(19) Europäisches Patentamt
European Patent Office
Office européen des brevets

(11) Veröffentlichungsnummer: **0 347 378**
**A1**

# (12) EUROPÄISCHE PATENTANMELDUNG

(21) Anmeldenummer: **89810423.7**

(22) Anmeldetag: **05.06.89**

(51) Int. Cl.⁴: **C 07 D 409/04**
A 01 N 43/50, C 07 D 333/78,
A 01 N 43/12

(30) Priorität: **13.06.88 CH 2251/88**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Schneider, Hans-Dieter, Dr.**
**Efringerstrasse 32**
**D-7858 Weil am Rhein (DE)**

(54) **Imidazol-Derivate.**

(57) Imidazol-Derivate der Formel I

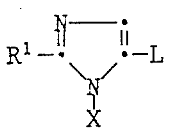

(I),

sowie stereochemisch isomere Formen davon und deren Salze, haben nützliche herbizide Eigenschaften. Die Substituenten $R^1$, L und X haben die hierin definierten Bedeutung.

EP 0 347 378 A1

**Beschreibung**

## Imidazol-Derivate

Die vorliegende Erfindung betrifft neue herbizid wirksame 5,6-Dihydrocyclopentathiophenyl-imidazol-Derivate, diese Wirkstoffe enthaltende agrochemische Mittel und ein Verfahren zur Bekämpfung von Unkräutern, vorzugsweise zur selektiven Bekämpfung von Unkräutern in Nutzpflanzenkulturen. Ferner betrifft die Erfindung auch ein Verfahren zur Herstellung der neuen Verbindungen, sowie neue Zwischenprodukte.

Die herbizid wirksamen 5,6-Dihydrocyclopentathiophenyl-imidazol-Derivate entsprechen der Formel I

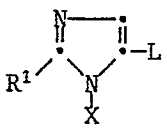 (I),

stereochemisch isomere Formen davon, sowie deren Salze, worin X einen Rest der Formel

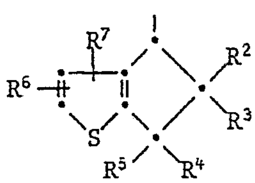 , 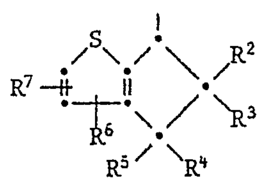 oder 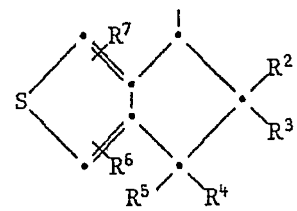

X1 X2 X3

$R^1$ Wasserstoff oder -SH, und

L Cyano, -COOH, $-COOR^8$, $-COSR^8$ $-CONR^9R^{10}$, $-CO-R^{11}$, $-CH_2-O-R^{12}$ oder eine Gruppe

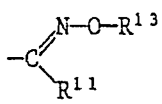 oder 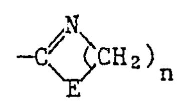

bedeuten, wobei

E für Sauerstoff, Schwefel, $-N(C_1-C_4Alkyl)-$ oder -NH- ,

n für die Zahl zwei oder drei,

$R^2$ und $R^3$ unabhängig voneinander für Wasserstoff, $C_1$-$C_4$-Alkyl, $C_2$-$C_4$-Alkenyl oder $C_2$-$C_4$-Alkinyl oder gemeinsam für eine spirocyclisch verknüpfte $C_2$-$C_6$-Alkylenkette,

$R^4$ und $R^5$ unabhängig voneinander für Wasserstoff oder $C_1$-$C_4$-Alkyl,

$R^6$ für Wasserstoff, Cyano, Halogen, $C_1$-$C_4$-Alkyl, $C_1$-$C_4$-Halogenalkyl, oder Nitro,

$R^7$ für Wasserstoff, Halogen oder $C_1$-$C_4$-Alkyl,

$R^8$ für $C_1$-$C_6$-Alkyl, $C_3$-$C_7$-Cycloalkyl, $C_2$-$C_6$-Alkoxyalkyl, Benzyl, $-CR^{14}R^{15}$-$C_2$-$C_4$-Alkenyl oder $-CR^{14}R^{15}$-$C_2$-$C_4$-Alkinyl,

$R^9$ für Wasserstoff, $C_1$-$C_4$-Alkyl, $C_1$-$C_4$-Alkoxy, Benzyl, $-CR^{14}R^{15}$-$C_2$-$C_4$-Alkenyl oder $-CR^{14}R^{15}$-$C_2$-$C_4$-Alkinyl,

$R^{10}$ für Wasserstoff oder $C_1$-$C_4$-Alkyl, oder

$R^9$ und $R^{10}$ zusammen mit dem sie tragenden Stickstoffatom für einen Pyrrolidin-, Piperidin- oder Morpholinrest,

$R^{11}$ für Wasserstoff, $C_1$-$C_6$-Alkyl, $C_3$-$C_7$-Cycloalkyl, $C_1$-$C_4$-Halogenalkyl oder gegebenenfalls durch $C_1$-$C_4$-Alkyl, $C_1$-$C_4$-Alkoxy, Nitro oder Halogen substituiertes Phenyl,

$R^{12}$ für Wasserstoff, $C_1$-$C_6$-Alkyl, gegebenenfalls durch $C_1$-$C_4$-Alkyl, $C_1$-$C_4$-Alkoxy, Halogen oder Nitro substituiertes Phenyl, $C_1$-$C_6$-Alkylcarbonyl, $C_1$-$C_4$-Halogenalkylcarbonyl oder gegebenenfalls durch $C_1$-$C_4$-Alkyl, $C_1$-$C_4$-Alkoxy, Halogen oder Nitro substituiertes Benzoyl,

$R^{13}$ für $C_1$-$C_4$-Alkyl, $-CR^{14}R^{15}$-$C_2$-$C_4$-Alkenyl, Benzyl oder $-CR^{14}R^{15}$-$C_2$-$C_4$-Chloralkenyl,

und

$R^{14}$ und $R^{15}$ unabhängig voneinander für Wasserstoff oder $C_1$-$C_4$-Alkyl stehen.

5-Imidazolcarbonsäure-Derivate mit verschiedenen carbocyclischen und heterocyclischen, kondensierten Ringsystemen als Substituenten in der Position 1 sind aus den Europäischen Patentanmeldungen EP-A-207 563 und EP-A-234 656 mit ihren biologischen Wirkungen sowie Verfahren zu ihrer Herstellung bekannt.

Ueberraschenderweise besitzen die Verbindungen der Formel I starke herbizide Eigenschaften, welche es erlauben, Unkräuter zu bekämpfen. Diese Eigenschaft gewinnt dadurch an Bedeutung, dass einige Nutzpflanzenkulturen nicht durch die Behandlung mit Verbindungen der Formel I geschädigt werden oder, dass nur bei sehr hohen Dosierungen geringfügige Schädigungen der Kultur auftreten. Somit sind die Verbindungen der Formel I wertvolle Selektivherbizide in Nutzpflanzenkulturen wie Getreide, Zuckerrüben,

Raps, Sojabohnen, Reis und Mais. Besonders in Reiskulturen kann ein breiter Bereich von Aufwandmengen angewendet werden, insbesondere dann, wenn es sich bei den Reiskulturen um verpflanzten Reis handelt und wenn die Verbindungen der Formel I nach der Verpflanzung ausgebracht werden.

Die aktiven Wirkstoffe der Formel I werden üblicherweise bei Aufwandmengen zwischen 0,01 und 5,0 kg Aktivsubstanz pro Hektar angewendet, um zufriedenstellende Resultate zu erzeugen. Wenn die Klima- und Bodenbedingungen es erfordern, können die Aufwandmengen in geeigneten Fällen die oben angegebenen Grenzwerte überschreiten. Die bevorzugten Aufwandmengen liegen jedoch im allgemeinen zwischen 0,02 kg und 1,0 kg Wirkstoff pro Hektar.

In den Definitionen ist unter Alkyl geradkettiges oder verzweigtes Alkyl zu verstehen; z.B. Methyl, Aethyl, n-Propyl, i-Propyl oder die vier isomeren Butyl. Unter Alkoxy ist zu verstehen: Methoxy, Aethoxy, Propyloxy oder die vier isomeren Butyloxy, insbesondere aber Methoxy, Aethoxy oder Isopropyloxy.

Halogen bedeutet in den obigen Definitionen Fluor, Chlor, Brom oder Jod, worunter Fluor und Chlor bevorzugt sind. Alkenyl bedeutet beispielsweise Vinyl, Allyl, 1-Methylvinyl, 3-Butenyl, 2-Butenyl, 1-Butenyl, 1-Propenyl, Methallyl oder 3-Methyl-2-butenyl, wobei Vinyl, Allyl und Methallyl bevorzugt sind. Beispiele für Alkinyl sind Aethinyl, 1-Propinyl, Propargyl, 1-butinyl, 2-Butinyl oder 3-Butinyl, vorzugsweise aber Aethinyl oder Propargyl. Cycloalkyl bedeutet im allgemeinen Cyclopropyl, Cyclobutyl, Cyclopentyl, Cyclohexyl oder Cycloheptyl, vorzugsweise Cyclopentyl oder Cyclohexyl. Reste, welche aus den gegebenen Bedeutungen kombiniert werden können, wie Halogenalkyl, Alkoxyalkyl, oder Halogenalkylcarbonyl haben die entsprechend kombinierten konkreten Gruppenmitglieder. Wichtige Beispiele dafür sind: Trifluormethyl, Chlormethyl, 2,2,2-Trifluoräthyl, Methoxymethyl, Methoxyäthyl, Aethoxyäthyl, Chlormethylcarbonyl, Trifluormethylcarbonyl oder Brommethylcarbonyl.

Die 5,6-Dihydrocyclopentathiophen-4-yl-Radikale X, welche über ein Stickstoffatom an den Imidazolring gebunden sind, umfassen folgende grundlegende Strukturen, die ihrerseits in insubstituierter Form oder mit den genannten Substituenten $R^2$, $R^3$, $R^4$, $R^5$, $R^6$ und $R^7$ vorliegen können:
5,6-Dihydro-4H-cyclopenta[b]thiophen-4-yl,
5,6-Dihydro-4H-cyclopenta[c]thiophen-4-yl, und
5,6-Dihydro-4H-cyclopenta[b]thiophen-6-yl.

Die Reste $R^2$ und $R^3$ sind an das gleiche Kohlenstoffatom gebunden. Somit können sie zusammen mit diesem Kohlenstoffatom einen spirocyclischen Ring bilden. Typische Vertreter solcher spirocyclischen Ringe sind Cyclopropan-, Cyclobutan-, Cyclopentan-, Cyclohexan- und Cycloheptan-Ringe.

Die Erfindung umfasst ebenfalls die Salze, die die Verbindungen der Formel I mit organischen oder anorganischen Basen wie Aminen, Alkalimetallbasen und Erdalkalimetallbasen oder quaternären Ammoniumbasen oder mit organischen oder anorganischen Säuren wie Mineralsäuren, Sulfonsäuren, Carbonsäuren oder phosphorhaltigen Säuren bilden können.

Beispiele salzbildender Mineralsäuren sind Fluorwasserstoffsäure, Chlorwasserstoffsäure, Bromwasserstoffsäure, Jodwasserstoffsäure, Schwefelsäure, Salpetersäure, Chlorsäure, Perchlorsäure oder Phosphorsäure. Bevorzugte salzbildende Sulfonsäuren sind Toluolsulfonsäuren, Benzolsulfonsäure, Methansulfonsäure oder Trifluormethansulfonsäure. Als salzbildende Carbonsäuren seien vorzugsweise Essigsäure, Trifluoressig säure, Benzoesäure, Chloressigsäure, Phthalsäure, Maleinsäure, Malonsäure und Zitronensäure genannt. Phosphor-haltige Säuren sind die verschiedenen Phosphonsäuren, phosphonigen Säuren und Phosphinsäuren.

Bevorzugte salzbildende Alkalimetallhydroxide und Erdalkalimetallhydroxide sind die Hydroxide von Lithium, Natrium, Kalium, Magnesium oder Calcium, insbesondere aber die von Natrium oder Kalium. Beispiele von geeigneten salzbildenden Aminen sind primäre, sekundäre und tertiäre aliphatische und aromatische Amine wie Methylamin, Aethylamin, Propylamin, Isopropylamin, die vier Butylaminisomeren, Dimethylamin Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und Isochinolin. Bevorzugte Amine sind Aethylamin, Propylamin, Diäthylamin ode Triäthylamin, insbesondere aber Isopropylamin, Diäthanolamin und 1,4-Diazabicyclo-[2.2.2]octan. Beispiele für quaternäre Ammoniumbasen sind im allgemeinen die Kationen von Halogenammoniumsalzen, beispielsweise das Tetramethylammoniumkation, das Trimethylbenzylammoniumkation, das Tetraäthylammoniumkation, das Trimethyläthylammoniumkation und auch das Ammoniumkation.

Die Erfindung umfasst ebenfalls alle optischen Isomeren der Verbindungen der Formel I. Diese können auftreten, wenn der Rest X in der Position 1 des Imidazolrings und/oder die Gruppe L der Verbindungen der Formel I asymmetrisch substituierte Kohlenstoffatome enthalten. Sofern keine nähere Angabe gemacht wird, umfasst die chemische Bezeichnung von Verbindungen deren Mischungen aller stereochemisch isomeren Formen. Die Mischungen enthalten alle Diastereomeren und Enantiomeren der zugrundeliegenden Molekülstruktur.

Die reinen isomeren Formen dieser optisch aktiven Verbindungen können aus den Mischungen durch übliche Trennungsmethoden erhalten werden. Wird im Einzelfall eine spezifische stereochemische Form gewünscht, so wird diese Verbindung vorzugsweise durch stereoselektive Syntheseverfahren hergestellt. In diesen Verfahren kommen mit Vorteil reine Formen der optisch aktiven Ausgangsmaterialien zur Anwendung.

Unter den Verbindungen der Formel I sind diejenigen bevorzugt, in denn entweder

a) $R^1$ für Wasserstoff steht, oder

b) L für $-COOR^8$, $-CONR^9R^{10}$ oder $-CO-R^{11}$ steht, oder

c) X für den Rest

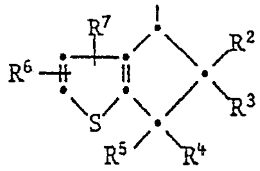

X1

steht, worin
$R^2$ und $R^3$ unabhängig voneinander Wasserstoff oder $C_1$-$C_4$-Alkyl, $R^4$ und $R^5$ Wasserstoff und $R^6$ Wasserstoff, $C_1$-$C_4$-Alkyl, oder Halogen bedeuten, oder

d) X für den Rest

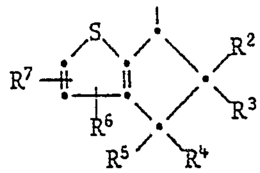

X2

steht, worin
$R^2$ und $R^3$ unabhängig voneinander Wasserstoff oder $C_1$-$C_4$-Alkyl, $R^4$ und $R^5$ Wasserstoff und $R^6$ Wasserstoff, $C_1$-$C_4$-Alkyl oder Halogen bedeuten.

Unter den Verbindungen der Gruppe b) sind solche Untergruppen bevorzugt, in denen entweder L für $C_1$-$C_4$-Alkoxycarbonyl oder für $C_1$-$C_4$-Alkylcarbonyl oder für -CO-$NR^9R^{10}$ steht, worin $R^9$ und $R^{10}$ unabhängig voneinander Wasserstoff oder $C_1$-$C_4$-Alkyl bedeuten. Besonders bevorzugt ist $C_1$-$C_4$-Alkoxycarbonyl.

Unter den Verbindungen der Gruppe c) sind diejenigen bevorzugt, worin $R^1$ für Wasserstoff und L für $C_1$-$C_4$-Alkoxycarbonyl, $C_1$-$C_4$-Alkylcarbonyl, Carbamoyl, Methylcarbamoyl oder Dimethylcarbamoyl stehen.

Unter den Verbindungen der Gruppe d) sind diejenigen bevorzugt, worin $R^1$ für Wasserstoff und L für $C_1$-$C_4$-Alkoxycarbonyl, $C_1$-$C_4$-Alkylcarbonyl, Carbamoyl, Methylcarbamoyl oder Dimethylcarbamoyl stehen.

Als bevorzugte Einzelverbindungen der vorliegenden Erfindung sind zu nennen:
1-(5,6-Dihydro-5,5-dimethyl-4H-cyclopenta[b]thiophen-4-yl)-5-imidazolcarbonsäure-methylester und
1-(5,6-Dihydro-5,5-dimethyl-4H-cyclopenta[b]thiophen-6-yl)-5-imidazolcarbonsäure-methylester.

Die erfindungsgemässen Verbindungen der Formel I werden im allgemeinen nach den folgenden Methoden hergestellt.

Nach einem ersten Verfahren erhält man Verbindungen der Formel I, worin $R^1$ für Wasserstoff steht, indem man ein Amin der Formel II

$X-NH_2$ (II)

worin X die unter Formel I gegebene Bedeutung hat, mit einem N-Cyan-Imidoameisensäureester der Formel III

R-O-CH=N-CN (III)

worin R für $C_1$-$C_4$-Alkyl steht, kondensiert, das entstehende N-Cyanoformamidin der Formel IV

X-NH-CH=N-CN (IV)

worin X die unter Formel I gegebene Bedeutung hat, mit einem Halogenessigsäureester der Formel V

$Hal-CH_2-L$ (V)

worin Hal für Chlor oder Brom und L für $C_1$-$C_6$-Alkoxycarbonyl stehen, in Gegenwart einer Base alkyliert, das entstehende Zwischenprodukt der Formel VI

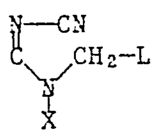

(VI)

worin X die unter Formel I gegebene Bedeutung hat und L für $C_1$-$C_6$-Alkoxycarbonyl steht, in Gegenwart einer Base zum 4-Amino-imidazol der Formel VII

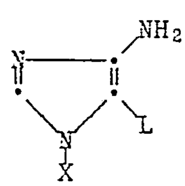

(VII)

worin X die unter Formel I gegebene Bedeutung hat und L für $C_1$-$C_6$-Alkoxycarbonyl steht, cyclisiert und diese Verbindung unter Diazotierung und Stickstoffabspaltung in das Produkt der Formel I, worin L für $C_1$-$C_6$-Alkoxycarbonyl und $R^1$ für Wasserstoff stehen, überführt und gewünschtenfalls die Gruppe L in 5-Stellung gemäss den übrigen Bedeutungen der Definition unter Formel I derivatisiert.

Der besondere Vorteil dieses Verfahrens ist in der Möglichkeit zu sehen, das Verfahren in einem einzigen Reaktionsgefäss - ohne Isolierung der Zwischenprodukte - durchführen zu können. Ferner können die ersten beiden Reaktionsschritte zur Alkylierung des primären Amins der Formel II mit den Produkten der Formeln III und V beliebig miteinander vertauscht werden.

Nach zweitem Verfahren erhält man die Verbindungen der Formel I, indem man das Amin der Formel II

$X-NH_2$ (II)

worin X die unter Formel I gegebene Bedeutung hat, mit einem Halogenessigsäureester der Formel V

$Hal-CH_2-L$ (V)

worin Hal für Chlor oder Brom und L für $C_1$-$C_6$-Alkoxycarbonyl stehen, in Gegenwart einer Base Alkyliert, den entstehenden Glycinester der Formel VIII

$X-NH-CH_2-L$ (VIII)

worin X die unter Formel I gegebene Bedeutung hat und L für $C_1$-$C_6$-Alkoxycarbonyl steht, in Gegenwart von Essigsäureanhydrid mit Ameisensäure formyliert, das entstehende Zwischenprodukt der Formel IX

$HCO-\underset{X}{N}-CH_2-L$ (IX)

worin X die unter Formel I gegebene Bedeutung hat und L für $C_1$-$C_6$-Alkoxycarbonyl steht, in Gegenwart einer Alkalibase mit einem Ameisensäure-$C_1$-$C_4$-alkylester umsetzt, das entstehende Zwischenprodukt der Formel X

$HCO-\underset{X}{N}-\underset{L}{C}=CH-O-M$ (X)

worin X die unter Formel I gegebene Bedeutung hat und L für $C_1$-$C_6$-Alkoxycarbonyl und M für ein Alkalikation steht, mit Thiocyansäure zum Produkt der Formel I, worin $R^1$ für -SH und L für $C_1$-$C_6$-Alkoxycarbonyl stehen, cyclisiert und diese Verbindung gewünschtenfalls in der 2-Stellung desulfuriert und gewünschtenfalls die Gruppe L in 5-Stellung gemäss den übrigen Bedeutungen der Definition unter Formel I derivatisiert.

Bei der Derivatisierung des $C_1$-$C_6$-Alkoxycarbonylrestes in 5-Stellung wird in bekannter Weise modifiziert, beispielsweise durch Verseifung, Veresterung, Umesterung, Amidierung, Umamidierung, Oximierung oder Reduktion. Gewünschtenfalls kann auch eine Salzbildung am basischen Stickstoffatom des Imidazolringes erfolgen.

Die Cyanoverbindung (L = -CN) erhält man, indem man das Carbonsäureamid (L = $-CONH_2$) dehydratisiert. Als Mittel hierzu kommen beispielsweise Phosphorpentachlorid, Phosphoroxychlorid, Thienylchlorid, Phosphorpentoxid und Acetanhydrid in Frage. Die Reaktionstemperatur hängt von der Wahl des Dehydratisierungsmittels ab und liegt im allgemeinen zwischen +20° C und +120° C. Gegebenenfalls können auch inerte organische Lösungsmittel zugesetzt werden. Die Ester, Thiolester oder Amidderivate (L = $-COOR^8$, $-COSR^8$, $-CONR^9R^{10}$) lassen sich aus den Carbonsäuren (L = -COOH) oder den daraus erhältlichen aktivierten Verbindungen wie Säurehalogeniden oder Anhydriden oder gemischten Anhydriden durch Umsetzung mit den entsprechenden Alkoholen, Thiolen oder Aminverbindungen herstellen. Ester und Thiolester lassen sich ausserdem auch durch Alkylierung der Carbonsäuren mit Halogenalkylverbindungen in Gegenwart von Basen erhalten. Die heterocyclischen Verbindungen der Formel I mit

L = 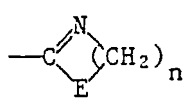

sind durch Umsetzung der entsprechenden Carbonsäuren, bzw. deren aktivierten Vertreter, wie ihren Säurehalogeniden, mit bifunktionellen Verbindungen wie Diaminen, Aminoalkohlen oder Aminothiolen nach an sich bekannten Verfahren zugänglich. Die Keton-Derivate (L = $-CO-R^{11}$ können aus den Estern durch Reduktion zur Aldehydstufe, und gewünschtenfalls Alkylierung zum sekundären Alkohol mittels Grignard-Addition und anschliessende Oxidation erhalten werden. Die Aether-Derivate (L = $-CH_2-O-R^{12}$) können aus den Estern durch Reduktion zu Alkohol und gewünschtenfalls angeschlossene Verätherung erhalten werden.

Die Zwischenprodukte der Formel VII sind neu. Sie werden speziell für die Synthese der Verbindungen der Formel I entwickelt. Sie sind daher Gegenstand der vorliegenden Erfindung. Die Amine der Formel II sind zum grossen Teil neu. Diese neuen Verbindungen der Formel II bilden ebenfalls einen Erfindungsgegenstand. Sie entsprechen der Formel II unter Ausnahme der Verbindung 4-Amino-5,6-dihydro-4H-cyclopenta[b]thiophen.

Die einzelnen Reaktionsstufen der verschiedenen Verfahren können mit Vorteil unter den anschliessend geschilderten Bedingungen durchgeführt werden.

Die Kondensation (II + III → IV) wird mit Vorteil in einem polaren Lösungsmittel wie Dimethylformamid oder Dimethylacetamid oder in einem Alkohol, dessen Alkylrest der Alkylgruppe des N-Cyan-imidoameisensäure esters der Formel III entspricht, bei einer Temperatur zwischen +20° C und +80° C durchgeführt. Im allgemeinen erfolgt die Reaktion spontan und ist exotherm.

Die Alkylierung des Produkts der Formel IV (IV + V → VI) erfolgt in Gegenwart einer Base als säurebindendes Mittel. Solche Reagenzien können sowohl anorganische als auch organische Basen sein.

Beispiele sind Alkalihydroxide, Alkalihydrogencarbonate, Alkalicarbonate, Erdalkalioxide, Erdalkalicarbonate, Alkalialkoholate oder tertiäre Amine, wie Natriumhydroxid, Kaliumhydroxid, Natriumcarbonat, Kaliumhydrogencarbonat, Magnesiumoxid, Calciumoxid, Natriummethylat, Natriumäthylat, Kaliummethylat, Kaliumäthylat, Kalium-tert.butylat, Pyridin, Triäthylamin etc. Die Reaktionstemperaturen liegen im allgemeinen zwischen 0°C und +80°C, vorzugsweise zwischen +10°C und +50°C. Sofern die Reaktion nicht lösungsmittelfrei durchgeführt werden soll, eignen sich insbesondere polare aprotische Lösungsmittel für die Alkylierung wie Dimethylformamid, Dimethylacetamid oder Dimethylsulfoxid.

Die cyclisierung des Zwischenprodukts der Formel VI zu den 4-Aminoimidazolen der Formel VII findet vorzugsweise unter Katalyse einer Base bei erhöhten Temperaturen statt. Geeignete Bedingungen liegen dann vor, wenn man in einem Alkohol, der dem zur Veresterung der Alkoxycarbonylgruppe verwendeten entspricht, in Gegenwart von katalytischen Mengen des durch Lösen eines Alkalimetalls erzeugten Alkoholats zum Sieden erhitzt. Andererseits lässt sich die Reaktion auch durch Erhitzen der Verbindung der Formel VI mit dem entsprechenden Alkoholat in einem polaren Lösungsmittel wie Dimethylformamid oder Dimethylsulfoxid herbeiführen. Die Reaktionstemperaturen liegen in der Regel zwischen +60°C und +140°C.

Die Desaminierung der 4-Stellung des Imidazolringes (VII → I) erfolgt zweckmässigerweise, indem man das 4-Amino-imidazol in Tetrahydrofuran, Dioxan oder Dimethylformamid mit tert.-Butylnitrit behandelt. Die Diazotierung der Aminogruppe und anschliessende Stickstoffabspaltung kann aber auch in wässrigem saurem Medium mit Natriumnitrit in Gegenwart von Salpetersäure, Salzsäure oder Schwefelsäure und angeschlossenem Verkochen mit hypophosphoriger Säure $H_3PO_2$ erfolgen.

Die Darstellung des Glycinesters der Formel VIII (II + V → VIII) wird unter den gleichen Reaktionsbedingungen durchgeführt wie der oben erläuterte Verfahrensschritt (IV + V → VI).

Die Formylierung des Glycinesters der Formel VIII unter Einsatz von Ameisensäure (VIII → IX) in Gegenwart eines wasserentziehenden Mittels wie Acetanhydrid erfolgt nach an sich für die Darstellung von Carbonsäureamiden üblichen Kondensationsbedingungen. Alternativ kann man die Formylierung auch durch Reaktion mit Ameisensäure in einer Wasserabscheidungsapparatur durch azeotrope Entfernung des Reaktionswassers erreichen.

Die Kondensation der Zwischenprodukte der Formel IX mit einem Ameisensäurealkylester in Gegenwart einer Alkalibase wird üblicherweise in einem inerten Lösungsmittel durchgeführt. Als Alkalibase haben sich Alkalialkoholate oder Alkalihydride erwiesen. Beispiele sind Natriummethylat, Kaliummethylat, Kaliumäthylat, Natriumäthylat, Natriumhydrid oder Lithiumhydrid. Inerte Lösungsmittel sind Alkohole wie Methanol, Aethanol oder Isopropanol oder Aether wie Diäthyläther, Tetrahydrofuran oder Dioxan. Die Reaktionstemperaturen liegen meistens zwischen -20°C und +60°C, vorzugsweise zwischen +10°C und +40°C.

Das Cyclisierungsverfahren der Enaminverbindung der Formel X zu den Produkten der Formel I wird zweckmässigerweise in einem wässrigen inerten Reaktionssystem, beispielsweise in Wasser oder in einer Mischung von Wasser mit Tetrahydrofuran oder Dioxan, bei Temperaturen zwischen +50°C und dem Siedepunkt des Lösungsmittels durchgeführt.

Die Desulfurierung der Produkte der Formel I, worin $R^1$ für -SH steht, kann beispielsweise durch Behandlung mit 15%iger salpetriger Säure bei Temperaturen zwischen +20°C und +40°C erfolgen oder durch Behandlung mit Nickel bei Temperaturen zwischen +50°C und +100°C.

Wird die Synthese von stereochemisch reinen Isomeren der Formel I beabsichtigt, ist es empfehlenswert, stereoselektive Reaktionsschritte und -bedingungen zu wählen. Andererseits können reine Isomeren häufig durch übliche Trennungsmethoden aus dem Isomerengemisch abgetrennt werden.

Die Ausgangsmaterialien der Formeln III und V sind bekannt, bzw. können analog zu bekannten Verbindungen hergestellt werden.

Die Herstellung der Amine der Formel II erfolgt durch Methoden, die in der Literatur bekannt sind, wie beispielsweise Reduktion von Iminen, Oximen oder Oximderivaten auf katalytischem oder elektrochemischem Wege oder mit Metallhydriden, wie Lithiumaluminiumhydrid oder Natriumborhydrid/Titantetrachlorid. Eine weitere Möglichkeit ist die reduktive Aminierung von Ketonen auf katalytischem Wege oder mit Hydriden wie Natriumcyanoborhydrid. Die Amine der Formel II kann man auch ausgehend von den Ketonen durch Reaktion mit Ammoniumformiat und anschliessender Hydrolyse der N-Formyl-Derivate gewinnen. Diese Reaktion ist in der Literatur als Leuckart-Wallach'sche Amin-Darstellung bekannt.

Die zur Herstellung der Amine der Formel II erforderlichen Oxime erfolgt durch an sich bekannte Umsetzung von entsprechenden Ketonen mit Hydroxylamin. Die Oxime der Formel XI, XII und XIII

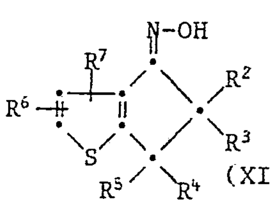 (XI) , 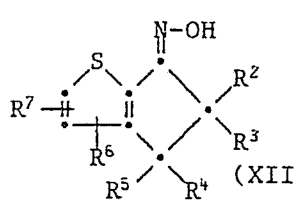 (XII) oder 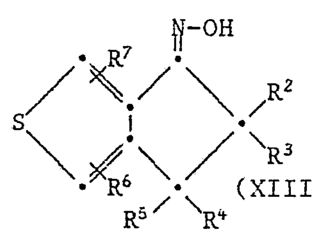 (XIII)

worin $R^2$, $R^3$, $R^4$, $R^5$, $R^6$ und $R^7$ die unter Formel I gegebenen Bedeutungen haben, sind zum grossen Teil neu. Diese neuen Verbindungen der Formeln XI, XII und XIII bilden einen weiteren Gegenstand der vorliegenden

Erfindung. Sie entsprechen der Formel XI unter Ausnahme der Verbindungen 5,6-Dihydro-5-methyl-4H-cyclopenta[b]thiophen-4-on-oxim und 5,6-Dihydro-2,3-dimethyl-4H-cyclopenta[b]thiophen-4-on-oxim; der Formel XII unter Ausnahme der Verbindungen 5,6-Dihydro-5-methyl-6H-cyclopenta[b]thiophen-6-on-oxim, 5,6-Dihydro-6H-cyclopenta[b]thiophen-6-on-oxim und 2-Chlor-5,6-dihydro-5-methyl-6H-cyclopenta[b]thiophen-6-on-oxim; und der Formel XIII unter Ausnahme der Verbindungen 5,6-Dihydro-1,3-dimethyl-4H-cyclopenta[c]thiophen-4-on-oxim, 1-Chlor-5,6-dihydro-4H-cyclopenta[c]thiophen-4-on-oxim, 5,6-Dihydro-1,3,5-trimethyl-4H-cyclopenta[c]thiophen-4-on-oxim und 1,3-Dichlor-5,6-dihydro-4H-cyclopenta[c]thiophen-4-on-oxim.

Die den Oximen der Formeln XI, XII und XIII zugrunde liegenden Ketone sind teilweise bekannt. Ebenso ist die Herstellung der Ketone teilweise in der Literatur beschrieben, wie zum Beispiel 5,6-Dihydro-4H-cyclopenta[b]thiophen-4-on (US 3,301,874), 5,6-Dihydro-5-methyl-4H-cyclopenta[b]thiophen-4-on (Acta Chem. Scand. 20, 1577 (1966)), 1,3-Dichlor-4-oxocyclopenta[c]-thiophen (J. Org. Chem. 32, 1226 (1967)) und 3-Methyl-5,6-dihydrocyclopenta[b]-thiophen-4-on (Bull. Chim. France 1054 (1966)).

Die neuen Ketone der Formeln XIV, XV und XVI bilden ebenfalls einen Gegenstand der vorliegenden Erfindung. Sie entsprechen den Formeln

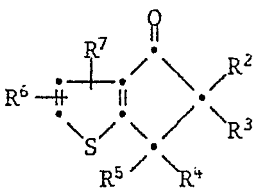

(XIV)

worin $R^2$, $R^3$, $R^4$, $R^5$, $R^6$ und $R^7$ die unter Formel I gegebenen Bedeutungen haben, mit der Massgabe, dass mindestens zwei der Reste $R^2$, $R^3$, $R^4$ und $R^5$ eine von Wasserstoff verschiedene Bedeutung haben;

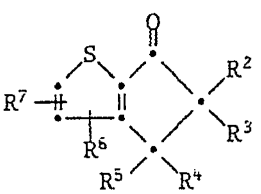

(XV)

worin $R^2$, $R^3$, $R^4$, $R^5$, $R^6$ und $R^7$ die unter Formel I gegebenen Bedeutungen haben mit der Massgabe, dass mindestens zwei der Reste $R^2$, $R^3$, $R^4$ und $R^5$ eine von Wasserstoff verschiedene Bedeutung haben, und Ausnahme der Verbindung 5,6-Dihydro-4,4-dimethyl-6H-cyclopenta[b]thiophen-6-on; und

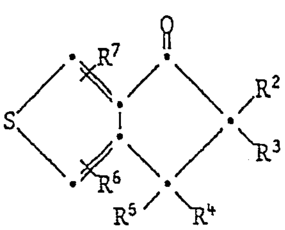

(XVI)

worin $R^2$, $R^3$, $R^4$, $R^5$, $R^6$ und $R^7$ die unter Formel I gegebenen Bedeutungen haben, mit der Massgabe, dass mindestens zwei der Reste $R^2$, $R^3$, $R^4$ und $R^5$ eine von Wasserstoff verschiedene Bedeutung haben.

Die neuen Ketone der Formeln XIV und XV werden beispielsweise analog zu den folgenden Reaktionsschemata erhalten.

Schema 1:

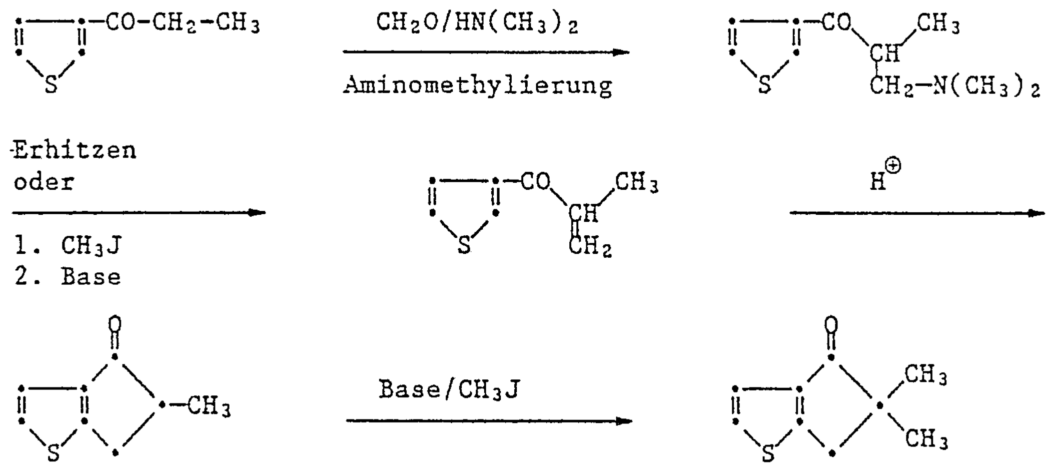


Schema 2:

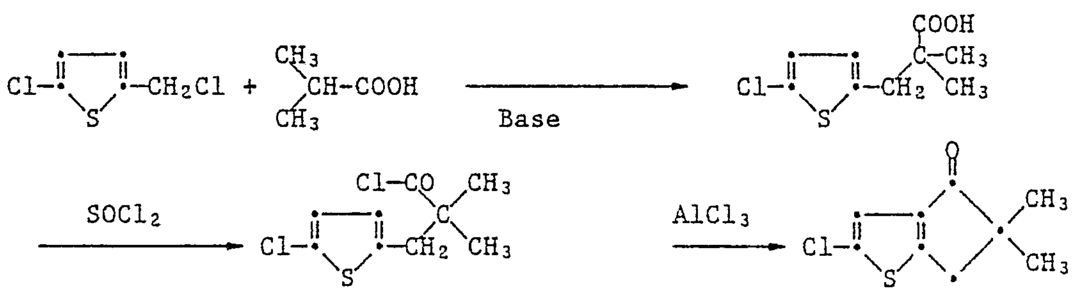


Die neuen Ketone der Formeln XVI werden beispielsweise analog zu den folgenden Reaktionsschemata erhalten.

Schema 3:

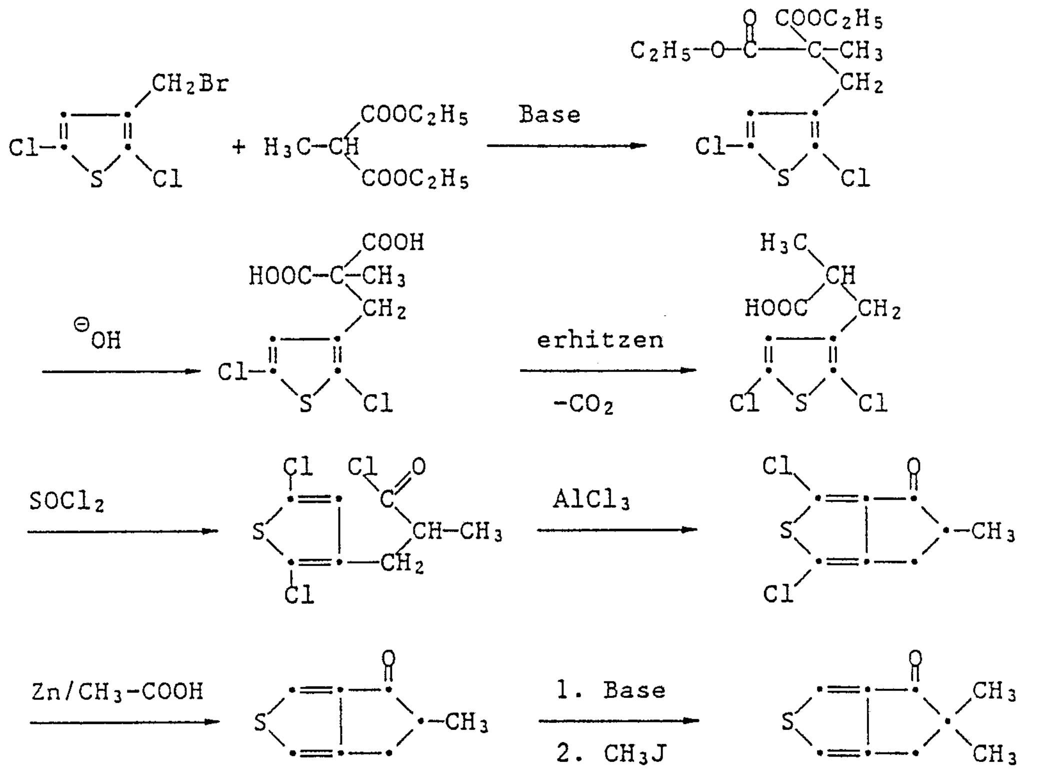


Die Verbindungen der Formel I sind stabil und erfordern keine besonderen Vorsichtsmassnahmen für ihre Handhabung.

Bei Anwendung der Verbindung der Formel I in dem angegebenen Bereich der Aufwandmengen zeichnen sich diese Wirkstoffe durch gute selektivherbizide Eigenschaften aus, die sie ausgezeichnet zum Einsatz in Kulturen von Nutzpflanzen insbesondere Zuckerrüben, Sojabohnen, Getreide und Mais und ganz besonders Reis befähigen. Teilweise werden dabei auch Unkräuter geschädigt, welchen bisher nur mit Totalherbiziden beizukommen war. Werden Aufwandmengen angewendet, die den empfohlenen Bereich weit übersteigen, so werden alle behandelten Pflanzen so stark in ihrer Entwicklung geschädigt, dass sie absterben.

Die Erfindung betrifft auch herbizide Mittel, welche die neuen Verbindungen der Formel I enthalten. Ebenso erstreckt sich die Erfindung auf Verfahren zur Bekämpfung von Unkräutern durch die Anwendung dieser neuen Wirkstoffe.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B.

Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien der Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:

| | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75%, vorzugsweise 10 bis 50 % |
| Wasser: | 94 bis 25 %, vorzugsweise 88 bis 30 % |
| oberflächenaktives Mittel: | 1 bis 40 %, vorzugsweise 2 bis 30 % |

Benetzbares Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 99 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die nachfolgenden Beispiele illustrieren die vorliegende Erfindung, ohne eine Begrenzung hiervon darzustellen. Die Herstellungsbeispiele zeigen, wie man die neuen Verbindungen der Formel I erhalten kann. Die biologischen Beispiele und die Formulierungsbeispiele demonstrieren die Anwendung der Wirkstoffe für agrochemische Zwecke.

Herstellungsbeispiele:

Beispiel H1: 1-(5,6-Dihydro-5,5-dimethyl-4H-cyclopenta[b]thiophen-4-yl)-5-imidazolcarbonsäuremethylester

a) 5,6-Dihydro-5,5-dimethyl-4H-cyclopenta[b]thiophen-4-on

Eine Lösung von 28,6 g 5,6-Dihydro-5-methyl-4H-cyclopenta[b]thiophen-4-on in 210 ml tert-butanol wird bei +25° bis +30°C zu einer Lösung von 23,1 g Kalium-tert-butylat in 250 ml tert-Butanol getropft. Die dunkelrot-braune Lösung wird für 1,5 Stunden gerührt. Danach lässt man 12,9 ml Methyljodid zulaufen. Nach Rühren für eine Stunde wird die rotbraune Suspension filtriert und das Filtrat eingedampft. Nach Destillation

des Rückstandes erhält man 26,0 g 5,6-Dihydro-5,5-dimethyl-4H-cyclopenta[b]thiophen-4-on als farbloses Oel vom Siedepunkt 43°C bei 0,03 mbar.

b) 4-Amino-5,6-dihydro-5,5-dimethyl-4H-cyclopenta[b]thiophen

In einem Druckreaktor werden 7,0 g 5,6-Dihydro-5,5-dimethyl-4H-cyclopenta[b]thiophen-4-on in einem Gemisch von 100 ml Tetrahydrofuran und 27 g flüssigen Ammoniak gelöst. Dieser Mischung werden 1,4 g eines feuchten sulfidierten 5 %igen Platin/Kohle-Katalysators zugefügt. Der Reaktor wird mit Wasserstoffgas bis zu einem Druck von 100 bar gefüllt und auf +175°C bis +180°C aufgeheizt. Nachdem die Hydrierung zum Stillstand gekommen ist, wird der Katalysator abfiltriert, das Filtrat eingeengt und der Rückstand im Kugelrohr destilliert. Man erhält 5,4 g 4-Amino-5,6-dihydro-5,5-dimethyl-4H-cyclopenta[b]thiophen vom Siedepunkt +80°C bei 0,08 mbar.

c) N-Cyano-N′-(5,6-dihydro-5,5-dimethyl-4H-cyclopenta[b]thiophen-4-yl)-formamidin

2,7 g N-Cyan-imidoameisensäure-äthylester lässt man zu einer Lösung von 3,0 g 4-Amino-5,6-dihydro-5,5-dimethyl-4H-cyclopenta[b]thiophen in 1,0 ml Aethanol zutropfen. Die exotherm verlaufende Reaktion ist nach 2 Stunden beendet. Das Reaktionsgemisch wird eingedampft. Der kristalline Rückstand wird aus Aether umkristallisiert. Man erhält 2,2 g N-Cyano-N′-(5,6-dihydro-5,5-dimethyl-4H-cyclopenta[b]thiophen-4-yl)-formamidin vom Schmelzpunkt 118-119°C.

d) 4-Amino-1-(5,6-dihydro-5,5-dimethyl-4H-cyclopenta[b]thiophen-4-yl)-5-imidazolcarbonsäure-methylester

Einer Mischung aus 6,2 g N-Cyano-N′-(5,6-dihydro-5,5-dimethyl-4H-cyclopenta[b]thiophen-4-yl)-formamidin und 5 ml Dimethylsulfoxid setzt man 3,3 g Kalium-tert-butylat zu. Nachdem 15 Stunden bei Raumtemperatur gerührt worden ist, lässt man 3,1 ml Bromessigsäuremethylester zutropfen. Nach 2 Stunden wird das Reaktionsgemisch mit Eiswasser versetzt. Extraktion mit Aether, Trocknen der organischen Phase mit Natriumsulfat und Abdampfen des Lösungsmittels liefert 8,4 g N-Cyano-N′-(5,6-dihydro-5,5-dimethyl-4H-cyclopenta[b]thiophen-4-yl)-N′-methoxycarbonylmethylformamidin als gelbbraunes Oel. Das Oel wird in 8 ml Methanol vorgelegt und nach Zusatz von 2,6 ml 30 %iger methanolischer Natriummethylat-Lösung für 16 Stunden zum Sieden erhitzt. Nach dem Abkühlen wird die Reaktionslösung auf ein Eiswasser/Aether-Gemisch gegossen. Die organische Phase wird abgetrennt, mit Wasser extrahiert und über Natriumsulfat getrocknet. Nach Filtrieren umd Abdampfen erhält man 5,1 g braune Kristalle, die an Kieselgel chromatographisch gereinigt werden (Eluationsmittel: Essigester/Hexan 1:1). Man erhält 3,7 g 4-Amino--1-(5,6-dihydro-5,5-dimethyl-4H-cyclopenta[b]thiophen-4-yl)-5-imidazolcarbonsäure-methylester in Form beiger Kristalle mit einem Schmelzpunkt von 161-162°C.

e) Einer Lösung von 0,5 ml tert-Butylnitrit in 4 ml Dimethylformamid setzt man 0,7 g 4-Amino-1-(5,6-dihydro-5,5-dimethyl-4H-cyclopenta[b]thiophen-4-yl)-5-imidazolcarbonsäure-methylester zu. Nachdem für 16 Stunden bei Raumtemperatur gerührt worden ist, wird das Reaktionsgemisch auf Eiswasser gegossen. Nach Extraktion mit Aether wird die organische Phase mit gesättigter Kochsalzlösung gewaschen, über Natriumsulfat getrocknet, filtriert und eingedampft. Der Rückstand wird an Kieselgel chromatographisch gereinigt (Eluationsmittel: Essigester/Hexan 1:1). Man erhält so 0,2 g 1-(5,6-Dihydro-5,5-dimethyl-4H-cyclopenta[b]thiophen-4-yl)-5-imidazolcarbonsäure-methylester vom Schmelzpunkt 62-63°C (Verbindung 1.14).

In analoger Weise werden die in den angeschlossenen Tabellen aufgelisteten Zwischen- und Endprodukte erhalten.

Tabelle 1:

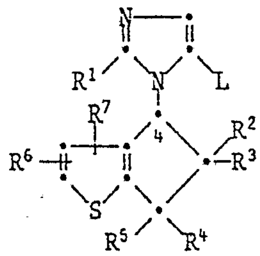

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 1.01 | $CO_2CH_3$ | H | H | H | H | H | H | H | |
| 1.02 | $CO_2CH_3$ | SH | H | H | H | H | H | H | |
| 1.03 | $CO_2CH_3$ | H | $CH_3$ | H | H | H | H | H | |
| 1.04 | $CO_2CH_3$ | H | $CH_3$ | H | H | H | H | H | 4,5-cis |
| 1.05 | $CO_2CH_3$ | H | $CH_3$ | H | H | H | H | H | 4,5-trans |
| 1.06 | $CO_2CH_3$ | H | $CH_3$ | H | $CH_3$ | H | H | H | |
| 1.07 | $CO_2CH_3$ | H | $CH_3$ | H | $CH_3$ | $CH_3$ | H | H | |
| 1.08 | $CO_2CH_3$ | H | $CH_3$ | H | H | H | 2-$CH_3$ | H | |
| 1.09 | $CO_2CH_3$ | H | $CH_3$ | H | H | H | 2-Cl | H | |
| 1.10 | $CO_2CH_3$ | H | $CH_3$ | H | H | H | 3-$CH_3$ | H | |
| 1.11 | $CO_2CH_3$ | H | $CH_3$ | H | H | H | 3-F | H | |
| 1.12 | $CO_2CH_3$ | H | $CH_3$ | H | H | H | 2-$CH_3$ | 3-Br | |
| 1.13 | $CO_2CH_3$ | H | $CH_3$ | H | H | H | 2-Cl | 3-Cl | |
| 1.14 | $CO_2CH_3$ | H | $CH_3$ | $CH_3$ | H | H | H | H | Smp. 60-61°C |
| 1.15 | $CO_2CH_3$ | SH | $CH_3$ | $CH_3$ | H | H | H | H | |
| 1.16 | $CO_2CH_3$ | H | $CH_3$ | $CH_3$ | $CH_3$ | H | H | H | |
| 1.17 | $CO_2CH_3$ | H | $CH_3$ | $CH_3$ | $CH_3$ | H | H | H | 4,6-cis |
| 1.18 | $CO_2CH_3$ | H | $CH_3$ | $CH_3$ | $CH_3$ | H | H | H | 4,6-trans |
| 1.19 | $CO_2CH_3$ | H | $CH_3$ | $CH_3$ | $CH_3$ | $CH_3$ | H | H | |
| 1.20 | $CO_2CH_3$ | H | $CH_3$ | $CH_3$ | H | H | 2-$CH_3$ | H | |
| 1.21 | $CO_2CH_3$ | H | $CH_3$ | $CH_3$ | H | H | 2-Cl | H | Smp.101-102°C |
| 1.22 | $CO_2CH_3$ | H | $CH_3$ | $CH_3$ | H | H | 2-$C_2H_5$ | H | |
| 1.23 | $CO_2CH_3$ | H | $CH_3$ | $CH_3$ | H | H | 2-$C(CH_3)_3$ | H | |
| 1.24 | $CO_2CH_3$ | H | $CH_3$ | $CH_3$ | H | H | 2-CN | H | |
| 1.25 | $CO_2CH_3$ | H | $CH_3$ | $CH_3$ | H | H | 2-$NO_2$ | H | |
| 1.26 | $CO_2CH_3$ | H | $CH_3$ | $CH_3$ | H | H | 2-$CH_3$ | 3-Br | |
| 1.27 | $CO_2CH_3$ | H | $CH_3$ | $CH_3$ | H | H | 2-$CH_3$ | 3-$CH_3$ | |
| 1.28 | $CO_2CH_3$ | H | $CH_3$ | $CH_3$ | H | H | 3-$CH_3$ | H | |
| 1.29 | $CO_2CH_3$ | H | $CH_3$ | $CH_3$ | H | H | 3-F | H | |
| 1.30 | $CO_2CH_3$ | H | $CH_3$ | $CH_3$ | H | H | 3-$NO_2$ | H | |
| 1.31 | $CO_2CH_3$ | H | $CH_3$ | $CH_3$ | H | H | 3-Cl | H | |
| 1.32 | $CO_2CH_3$ | SH | $-CH_2-CH_2-$ | | H | H | H | H | |
| 1.33 | $CO_2CH_3$ | H | $-CH_2CH_2-$ | | H | H | H | H | |
| 1.34 | $CO_2CH_3$ | H | $-CH_2CH_2-$ | | H | H | 2-$CH_3$ | H | |
| 1.35 | $CO_2CH_3$ | H | $-CH_2CH_2-$ | | H | H | 2-Cl | H | |
| 1.36 | $CO_2CH_3$ | H | H | H | $CH_3$ | H | H | H | |

Tabelle 1: (Fortsetzung)

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 1.37 | $CO_2CH_3$ | H | H | H | $CH_3$ | $CH_3$ | H | H | |
| 1.38 | $CO_2CH_3$ | H | $C_2H_5$ | H | H | H | H | H | |
| 1.39 | $CO_2CH_3$ | H | $C_2H_5$ | H | H | H | H | H | 4,5-cis |
| 1.40 | $CO_2CH_3$ | H | $C_2H_5$ | H | H | H | H | H | 4,5-trans |
| 1.41 | $CO_2CH_3$ | H | $C_2H_5$ | $CH_3$ | H | H | H | H | |
| 1.42 | $CO_2CH_3$ | H | $C_2H_5$ | $C_2H_5$ | H | H | H | H | |
| 1.43 | $CO_2CH_3$ | H | $C_2H_5$ | $C_2H_5$ | H | H | 2-$CH_3$ | H | |
| 1.44 | $CO_2CH_3$ | H | $C_2H_5$ | $C_2H_5$ | H | H | 3-$CH_3$ | H | |
| 1.45 | $CO_2CH_3$ | H | $C_2H_5$ | $C_2H_5$ | H | H | 2-Cl | H | |
| 1.46 | $CO_2CH_3$ | H | $C_2H_5$ | $C_2H_5$ | H | H | 3-Cl | 3-$CH_3$ | |
| 1.47 | $CO_2CH_3$ | SH | $C_2H_5$ | $C_2H_5$ | H | H | H | H | |
| 1.48 | $CO_2CH_3$ | H | $C_2H_5$ | $C_2H_5$ | $CH_3$ | H | H | H | |
| 1.49 | $CO_2CH_3$ | H | $C_3H_7$-n | H | H | H | H | H | |
| 1.50 | $CO_2CH_3$ | H | $C_3H_7$-n | H | H | H | H | H | |
| 1.51 | $CO_2CH_3$ | H | $-(CH_2)_4-$ | | H | H | H | H | |
| 1.52 | $CO_2CH_3$ | H | $-(CH_2)_4-$ | | $CH_3$ | H | H | H | |
| 1.53 | $CO_2CH_3$ | H | $-(CH_2)_4-$ | | H | H | 2-Cl | H | |
| 1.54 | $CO_2CH_3$ | H | $-(CH_2)_4-$ | | H | H | 2-$CH_3$ | H | |
| 1.55 | $CO_2CH_3$ | H | $-(CH_2)_4-$ | | H | H | 3-$CH_3$ | H | |
| 1.56 | $CO_2CH_3$ | H | $-CH_2CH=CH_2$ | H | H | H | H | H | |
| 1.57 | $CO_2CH_3$ | H | $CH_2CH=CH_2$ | $CH_3$ | H | H | H | H | |
| 1.58 | $CO_2CH_3$ | H | $C_4H_9$-n | H | H | H | H | H | |
| 1.59 | $CO_2C_2H_5$ | H | $CH_3$ | H | H | H | H | H | |
| 1.60 | $CO_2C_2H_5$ | H | $CH_3$ | H | $CH_3$ | H | H | H | |
| 1.61 | $CO_2C_2H_5$ | H | $CH_3$ | $CH_3$ | H | H | H | H | |
| 1.62 | $CO_2C_2H_5$ | H | $CH_3$ | $CH_3$ | $CH_3$ | H | H | H | |
| 1.63 | $CO_2C_2H_5$ | SH | $CH_3$ | $CH_3$ | H | H | H | H | |
| 1.64 | $CO_2C_2H_5$ | H | $CH_3$ | $CH_3$ | H | H | 2-$CH_3$ | H | |
| 1.65 | $CO_2C_2H_5$ | H | $CH_3$ | $CH_3$ | H | H | 2-Cl | H | |
| 1.66 | $CO_2C_2H_5$ | H | $CH_3$ | $CH_3$ | H | H | 2-CN | H | |
| 1.67 | $CO_2C_2H_5$ | H | $CH_3$ | $CH_3$ | H | H | 2-$NO_2$ | H | |
| 1.68 | $CO_2C_2H_5$ | H | $CH_3$ | $CH_3$ | H | H | 2-$CH_3$ | 3-Cl | |
| 1.69 | $CO_2C_2H_5$ | H | $CH_3$ | $CH_3$ | H | H | 2-$CH_3$ | 3-$CH_3$ | |
| 1.70 | $CO_2C_2H_5$ | H | $CH_3$ | $CH_3$ | H | H | 2-Cl | 3-$CH_3$ | |
| 1.71 | $CO_2C_2H_5$ | H | $CH_3$ | $CH_3$ | H | H | H | F | |
| 1.72 | $CO_2C_2H_5$ | H | $-CH_2CH_2-$ | | H | H | H | H | |
| 1.73 | $CO_2C_2H_5$ | H | $-CH_2CH_2-$ | | H | H | 2-Cl | H | |
| 1.74 | $CO_2C_2H_5$ | H | $-CH_2CH_2-$ | | H | H | 2-$CH_3$ | H | |

Tabelle 1: (Fortsetzung)

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 1.75 | $CO_2C_2H_5$ | H | $C_2H_5$ | H | H | H | H | H | |
| 1.76 | $CO_2C_2H_5$ | H | $C_2H_5$ | $C_2H_5$ | H | H | H | H | |
| 1.77 | $COCH_3$ | H | $CH_3$ | $CH_3$ | H | H | H | H | |
| 1.78 | $COCH_3$ | H | $CH_3$ | $CH_3$ | H | H | 2-$CH_3$ | H | |
| 1.79 | $COCH_3$ | H | $CH_3$ | $CH_3$ | H | H | 2-Cl | H | |
| 1.80 | $COCH_3$ | H | $-CH_2CH_2-$ | | H | H | H | H | |
| 1.81 | $COCH_3$ | H | $C_2H_5$ | H | H | H | H | H | |
| 1.82 | $COCH_3$ | H | $C_2H_5$ | $C_2H_5$ | H | H | H | H | |
| 1.83 | $COC_2H_5$ | H | $CH_3$ | $CH_3$ | H | H | H | H | |
| 1.84 | $COC_2H_5$ | H | $-CH_2CH_2-$ | | H | H | H | H | |
| 1.85 | $CONH_2$ | H | $CH_3$ | $CH_3$ | H | H | H | H | |
| 1.86 | $CONH_2$ | H | $C_2H_5$ | H | H | H | H | H | |
| 1.87 | $CONH_2$ | H | $C_2H_5$ | $C_2H_5$ | H | H | H | H | |
| 1.88 | $CONHCH_3$ | H | $CH_3$ | $CH_3$ | H | H | H | H | |
| 1.89 | $CON(CH_3)_2$ | H | $CH_3$ | $CH_3$ | H | H | H | H | |
| 1.90 | CN | H | $CH_3$ | $CH_3$ | H | H | H | H | |
| 1.91 | $CO_2CH_2CH{=}CH_2$ | H | $CH_3$ | $CH_3$ | H | H | H | H | |
| 1.92 | $CO_2CH_2C{\equiv}CH$ | H | $CH_3$ | $CH_3$ | H | H | H | H | |
| 1.93 | $CONHOCH_3$ | H | $CH_3$ | $CH_3$ | H | H | H | H | |

Tabelle 2:

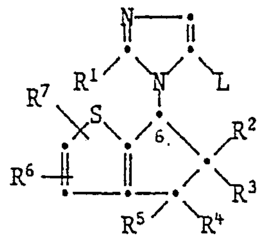

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 2.01 | $CO_2CH_3$ | H | H | H | H | H | H | H | |
| 2.02 | $CO_2CH_3$ | SH | H | H | H | H | H | H | |
| 2.03 | $CO_2CH_3$ | H | $CH_3$ | H | H | H | H | H | Smp. 55-58°C |
| 2.04 | $CO_2CH_3$ | H | $CH_3$ | H | H | H | H | H | 5,6-cis |
| 2.05 | $CO_2CH_3$ | H | $CH_3$ | H | H | H | H | H | 5,6-trans |
| 2.06 | $CO_2CH_3$ | H | $CH_3$ | H | $CH_3$ | H | H | H | |
| 2.07 | $CO_2CH_3$ | H | $CH_3$ | H | $CH_3$ | $CH_3$ | H | H | |
| 2.08 | $CO_2CH_3$ | H | $CH_3$ | H | H | H | 2-$CH_3$ | H | |
| 2.09 | $CO_2CH_3$ | H | $CH_3$ | H | H | H | 2-Cl | H | |
| 2.10 | $CO_2CH_3$ | H | $CH_3$ | H | H | H | 2-$NO_2$ | H | |
| 2.11 | $CO_2CH_3$ | H | $CH_3$ | H | H | H | 3-Br | 2-$CH_3$ | |
| 2.12 | $CO_2CH_3$ | H | $CH_3$ | H | H | H | 2-Cl | 3-Cl | |
| 2.13 | $CO_2CH_3$ | H | $CH_3$ | $CH_3$ | H | H | H | H | Smp.111-112°C |
| 2.14 | $CO_2CH_3$ | SH | $CH_3$ | $CH_3$ | H | H | H | H | |
| 2.15 | $CO_2CH_3$ | H | $CH_3$ | $CH_3$ | $CH_3$ | H | H | H | |
| 2.16 | $CO_2CH_3$ | H | $CH_3$ | $CH_3$ | $CH_3$ | H | H | H | 4,6-cis |
| 2.17 | $CO_2CH_3$ | H | $CH_3$ | $CH_3$ | $CH_3$ | H | H | H | 4,6-trans |
| 2.18 | $CO_2CH_3$ | H | $CH_3$ | $CH_3$ | $CH_3$ | $CH_3$ | H | H | |
| 2.19 | $CO_2CH_3$ | H | $CH_3$ | $CH_3$ | H | H | 2-$CH_3$ | H | |
| 2.20 | $CO_2CH_3$ | H | $CH_3$ | $CH_3$ | H | H | 2-Cl | H | |
| 2.21 | $CO_2CH_3$ | H | $CH_3$ | $CH_3$ | H | H | 2-$NO_2$ | H | |
| 2.22 | $CO_2CH_3$ | H | $CH_3$ | $CH_3$ | H | H | 2-CN | H | |
| 2.23 | $CO_2CH_3$ | H | $CH_3$ | $CH_3$ | H | H | 2-$C_4H_9$-t | H | |
| 2.24 | $CO_2CH_3$ | H | $CH_3$ | $CH_3$ | H | H | 3-Br | 2-$CH_3$ | |
| 2.25 | $CO_2CH_3$ | H | $CH_3$ | $CH_3$ | H | H | 2-Cl | 3-Cl | |
| 2.26 | $CO_2CH_3$ | H | $CH_3$ | $CH_3$ | H | H | 2-$NO_2$ | 3-$CH_3$ | |
| 2.27 | $CO_2CH_3$ | H | $CH_3$ | $CH_3$ | H | H | 3-$CH_3$ | H | |
| 2.28 | $CO_2CH_3$ | H | $CH_3$ | $CH_3$ | H | H | 3-CN | H | |
| 2.29 | $CO_2CH_3$ | H | $-CH_2CH_2-$ | | H | H | H | H | |
| 2.30 | $CO_2CH_3$ | SH | $-CH_2CH_2-$ | | H | H | H | H | |
| 2.31 | $CO_2CH_3$ | H | $-CH_2CH_2-$ | | H | H | 2-$CH_3$ | H | |
| 2.32 | $CO_2CH_3$ | H | $-CH_2CH_3-$ | | H | H | 2-Cl | H | |
| 2.33 | $CO_2CH_3$ | H | $-CH_2CH_2-$ | | H | H | 2-$NO_2$ | H | |
| 2.34 | $CO_2CH_3$ | H | H | H | $CH_3$ | H | H | H | |
| 2.35 | $CO_2CH_3$ | H | H | H | $CH_3$ | $CH_3$ | H | H | |
| 2.36 | $CO_2CH_3$ | H | $C_2H_5$ | H | H | H | H | H | |
| 2.37 | $CO_2CH_3$ | H | $C_2H_5$ | H | H | H | H | H | 5,6-cis |
| 2.38 | $CO_2CH_3$ | H | $C_2H_5$ | H | H | H | H | H | 5,6-trans |

Tabelle 2 (Fortsetzung)

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 2.39 | $CO_2CH_3$ | H | $C_2H_5$ | $CH_3$ | H | H | H | H | |
| 2.40 | $CO_2CH_3$ | H | $C_2H_5$ | $C_2H_5$ | H | H | H | H | |
| 2.41 | $CO_2CH_3$ | H | $C_2H_5$ | $C_2H_5$ | H | H | 2-$CH_3$ | H | |
| 2.42 | $CO_2CH_3$ | H | $C_2H_5$ | $C_2H_5$ | H | H | 2-Cl | H | |
| 2.43 | $CO_2CH_3$ | H | $C_2H_5$ | $C_2H_5$ | H | H | 3-$CH_3$ | 2-Cl | |
| 2.44 | $CO_2CH_3$ | SH | $C_2H_5$ | $C_2H_5$ | H | H | H | H | |
| 2.45 | $CO_2CH_3$ | H | $C_2H_5$ | $C_2H_5$ | $CH_3$ | H | H | H | |
| 2.46 | $CO_2CH_3$ | H | $C_3H_7$-n | H | H | H | H | H | |
| 2.47 | $CO_2CH_3$ | H | $C_3H_7$-i | H | H | H | H | H | |
| 2.48 | $CO_2CH_3$ | H | $-(CH_2)_4-$ | | H | H | H | H | |
| 2.49 | $CO_2CH_3$ | H | $-(CH_2)_4-$ | | $CH_3$ | H | H | H | |
| 2.50 | $CO_2CH_3$ | H | $-(CH_2)_4-$ | | H | H | 2-$CH_3$ | H | |
| 2.51 | $CO_2CH_3$ | H | $-(CH_2)_4-$ | | H | H | 2-Cl | H | |
| 2.52 | $CO_2CH_3$ | H | $-CH_2CH=CH_2$ | H | H | H | H | H | |
| 2.53 | $CO_2CH_3$ | H | $CH_2C\equiv CH$ | H | H | H | H | H | |
| 2.54 | $CO_2CH_3$ | H | $C_4H_9$-n | H | H | H | H | H | |
| 2.55 | $CO_2C_2H_5$ | H | $CH_3$ | H | H | H | H | H | |
| 2.56 | $CO_2C_2H_5$ | H | $CH_3$ | H | H | H | H | H | 5,6-cis |
| 2.57 | $CO_2C_2H_5$ | H | $CH_3$ | H | H | H | H | H | 5,6-trans |
| 2.58 | $CO_2C_2H_5$ | H | $CH_3$ | $CH_3$ | H | H | H | H | |
| 2.59 | $CO_2C_2H_5$ | H | $CH_3$ | $CH_3$ | $CH_3$ | H | H | H | |
| 2.60 | $CO_2C_2H_5$ | H | $CH_3$ | $CH_3$ | $CH_3$ | $CH_3$ | H | H | |
| 2.61 | $CO_2C_2H_5$ | H | $CH_3$ | $CH_3$ | H | H | 2-$CH_3$ | H | |
| 2.62 | $CO_2C_2H_5$ | H | $CH_3$ | $CH_3$ | H | H | 2-Cl | H | |
| 2.63 | $CO_2C_2H_5$ | H | $CH_3$ | $CH_3$ | H | H | 2-$NO_2$ | H | |
| 2.64 | $CO_2C_2H_5$ | H | $CH_3$ | $CH_3$ | H | H | 2-CN | H | |
| 2.65 | $CO_2C_2H_5$ | H | $CH_3$ | $CH_3$ | H | H | 2-$C_4H_9$-t | H | |
| 2.66 | $CO_2C_2H_5$ | H | $CH_3$ | $CH_3$ | H | H | 3-Cl | 2-$CH_3$ | |
| 2.67 | $CO_2C_2H_5$ | H | $CH_3$ | $CH_3$ | H | H | 3-$CH_3$ | 2-Cl | |
| 2.68 | $CO_2C_2H_5$ | H | $CH_3$ | $CH_3$ | H | H | 3-$CH_3$ | H | |
| 2.69 | $CO_2C_2H_5$ | H | $CH_3$ | $CH_3$ | H | H | 2-F | H | |
| 2.70 | $CO_2C_2H_5$ | H | $-CH_2CH_2-$ | | H | H | H | H | |
| 2.71 | $CO_2C_2H_5$ | H | $-CH_2CH_2-$ | | H | H | 2-$CH_3$ | H | |
| 2.72 | $CO_2C_2H_5$ | H | $-CH_2CH_2-$ | | H | H | 2-Cl | H | |
| 2.73 | $CO_2C_2H_5$ | H | $C_2H_5$ | H | H | H | H | H | |
| 2.74 | $CO_2C_2H_5$ | H | $C_2H_5$ | $C_2H_5$ | H | H | H | H | |
| 2.75 | $COCH_3$ | H | $CH_3$ | $CH_3$ | H | H | H | H | |
| 2.76 | $COCH_3$ | H | $CH_3$ | $CH_3$ | H | H | 2-$CH_3$ | H | |

Tabelle 2: (Fortsetzung)

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 2.77 | $COCH_3$ | H | $CH_3$ | $CH_3$ | H | H | 2-Cl | H | |
| 2.78 | $COCH_3$ | H | $CH_3$ | $CH_3$ | H | H | 2-$CH_3$ | H | |
| 2.79 | $COCH_3$ | H | $-CH_2CH_2-$ | | H | H | H | H | |
| 2.80 | $COCH_3$ | H | $C_2H_5$ | H | H | H | H | H | |
| 2.81 | $COCH_3$ | H | $C_2H_5$ | H | H | H | H | H | |
| 2.82 | $COCH_3$ | H | $CH_2CH{=}CH_2$ | H | H | H | H | H | |
| 2.83 | $COC_2H_5$ | H | $CH_3$ | $CH_3$ | H | H | H | H | |
| 2.84 | $COC_2H_5$ | H | $-CH_2CH_2-$ | | H | H | H | H | |
| 2.85 | $CONH_2$ | H | $CH_3$ | $CH_3$ | H | H | H | H | |
| 2.86 | $CONH_2$ | H | $C_2H_5$ | H | H | H | H | H | |
| 2.87 | $CONHCH_3$ | H | $CH_3$ | $CH_3$ | H | H | H | H | |
| 2.88 | $CONHCH_3$ | H | $CH_3$ | $CH_3$ | H | H | 2-Cl | H | |
| 2.89 | $CON(CH_3)_2$ | H | $CH_3$ | $CH_3$ | H | H | H | H | |
| 2.90 | $CON(CH_3)_2$ | H | $CH_3$ | $CH_3$ | H | H | 2-Cl | H | |
| 2.91 | CN | H | $CH_3$ | $CH_3$ | H | H | H | H | |
| 2.92 | CN | H | $CH_3$ | $CH_3$ | H | H | 2-$CH_3$ | H | |
| 2.93 | $CO_2CH_2CH{=}CH_2$ | H | $CH_3$ | $CH_3$ | H | H | H | H | |
| 2.94 | $CO_2CH_2C{\equiv}CH$ | H | $CH_3$ | $CH_3$ | H | H | H | H | |
| 2.95 | $CONHOCH_3$ | H | $CH_3$ | $CH_3$ | H | H | H | H | |

Tabelle 3:

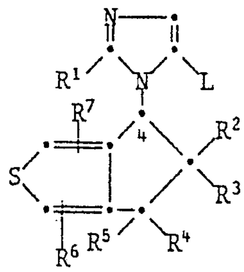

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 3.01 | $CO_2CH_3$ | H | H | H | H | H | H | H | |
| 3.02 | $CO_2CH_3$ | H | H | H | H | H | 1-Cl | 3-Cl | |
| 3.03 | $CO_2CH_3$ | H | H | H | H | H | 3-Cl | H | |
| 3.04 | $CO_2CH_3$ | H | H | H | H | H | 1-Cl | H | |
| 3.05 | $CO_2CH_3$ | H | H | H | H | H | 1-$CH_3$ | 3-$CH_3$ | |
| 3.06 | $CO_2CH_3$ | H | $CH_3$ | H | H | H | H | H | |
| 3.07 | $CO_2CH_3$ | H | $CH_3$ | H | H | H | H | H | 4,5-cis |
| 3.08 | $CO_2CH_3$ | H | $CH_3$ | H | H | H | H | H | 4,5-trans |
| 3.09 | $CO_2CH_3$ | H | $CH_3$ | H | H | H | 1-Cl | H | |
| 3.10 | $CO_2CH_3$ | H | $CH_3$ | H | H | H | 3-Cl | H | |
| 3.11 | $CO_2CH_3$ | H | $CH_3$ | H | H | H | 1-Cl | 3-Cl | |
| 3.12 | $CO_2CH_3$ | H | $CH_3$ | H | H | H | 1-$CH_3$ | 3-$CH_3$ | |
| 3.13 | $CO_2CH_3$ | H | $CH_3$ | H | $CH_3$ | H | H | H | |
| 3.14 | $CO_2CH_3$ | H | $CH_3$ | $CH_3$ | H | H | H | H | |
| 3.15 | $CO_2CH_3$ | SH | $CH_3$ | $CH_3$ | H | H | H | H | |
| 3.16 | $CO_2CH_3$ | H | $CH_3$ | $CH_3$ | H | H | 1-Cl | H | |
| 3.17 | $CO_2CH_3$ | H | $CH_3$ | $CH_3$ | H | H | 3-Cl | H | |
| 3.18 | $CO_2CH_3$ | H | $CH_3$ | $CH_3$ | H | H | 1-Cl | 3-Cl | |
| 3.19 | $CO_2CH_3$ | H | $CH_3$ | $CH_3$ | H | H | 1-$CH_3$ | 3-$CH_3$ | |
| 3.20 | $CO_2CH_3$ | H | $CH_3$ | $CH_3$ | H | H | 3-$CH_3$ | H | |
| 3.21 | $CO_2CH_3$ | H | $CH_3$ | $CH_3$ | H | H | 1-$NO_2$ | H | |
| 3.22 | $CO_2CH_3$ | H | $CH_3$ | $CH_3$ | $CH_3$ | H | H | H | |
| 3.23 | $CO_2CH_3$ | H | $CH_3$ | $CH_3$ | $CH_3$ | H | H | H | 4,6-cis |
| 3.24 | $CO_2CH_3$ | H | $CH_3$ | $CH_3$ | $CH_3$ | H | H | H | 4,6-trans |
| 3.25 | $CO_2CH_3$ | H | $CH_3$ | $CH_3$ | $CH_3$ | H | 1-Cl | H | |
| 3.26 | $CO_2CH_3$ | H | $CH_3$ | $CH_3$ | $CH_3$ | $CH_3$ | H | H | |
| 3.27 | $CO_2CH_3$ | H | $CH_3$ | $CH_3$ | $CH_3$ | $CH_3$ | 1-Cl | H | |
| 3.28 | $CO_2CH_3$ | H | $-CH_2CH_2-$ | | H | H | H | H | |
| 3.29 | $CO_2CH_3$ | SH | $-CH_2CH_2-$ | | H | H | H | H | |
| 3.30 | $CO_2CH_3$ | H | $-CH_2CH_2-$ | | H | H | 1-Cl | H | |
| 3.31 | $CO_2CH_3$ | H | $-CH_2CH_2-$ | | H | H | 3-Cl | H | |
| 3.32 | $CO_2CH_3$ | H | $-CH_2CH_2-$ | | H | H | 1-Cl | 3-Cl | |
| 3.33 | $CO_2CH_3$ | H | H | H | $CH_3$ | H | H | H | |
| 3.34 | $CO_2CH_3$ | H | $C_2H_5$ | H | H | H | H | H | |
| 3.35 | $CO_2CH_3$ | H | $C_2H_5$ | H | H | H | H | H | 4,5-cis |
| 3.36 | $CO_2CH_3$ | H | $C_2H_5$ | H | H | H | H | H | 4,5-trans |

Tabelle 3 (Fortsetzung)

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 3.37 | $CO_2CH_3$ | H | $C_2H_5$ | $C_2H_5$ | H | H | H | H | |
| 3.38 | $CO_2CH_3$ | H | $C_2H_5$ | $C_2H_5$ | H | H | 1-Cl | H | |
| 3.39 | $CO_2CH_3$ | H | $C_2H_5$ | $C_2H_5$ | H | H | 3-Cl | H | |
| 3.40 | $CO_2CH_3$ | H | $C_2H_5$ | $C_2H_5$ | H | H | 1-Cl | 3-Cl | |
| 3.41 | $CO_2CH_3$ | H | $C_3H_7$-i | H | H | H | H | H | |
| 3.42 | $CO_2CH_3$ | H | $-(CH_2)_4-$ | | H | H | H | H | |
| 3.43 | $CO_2CH_3$ | H | $-(CH_2)_4-$ | | H | H | 1-Cl | 3-Cl | |
| 3.44 | $CO_2CH_3$ | H | $CH_2CH{=}CH_2$ | H | H | H | H | H | |
| 3.45 | $CO_2CH_3$ | H | $CH_2CH{=}CH_2$ | $CH_3$ | H | H | H | H | |
| 3.46 | $CO_2CH_3$ | H | $C_4H_9$-n | H | H | H | H | H | |
| 3.47 | $CO_2C_2H_5$ | H | $CH_3$ | H | H | H | 1-Cl | H | |
| 3.48 | $CO_2C_2H_5$ | H | $CH_3$ | H | H | H | 3-Cl | H | |
| 3.49 | $CO_2C_2H_5$ | H | $CH_3$ | H | H | H | 1-Cl | 3-Cl | |
| 3.50 | $CO_2C_2H_5$ | H | $CH_3$ | $CH_3$ | H | H | H | H | |
| 3.51 | $CO_2C_2H_5$ | H | $CH_3$ | $CH_3$ | H | H | 1-Cl | H | |
| 3.52 | $CO_2C_2H_5$ | H | $CH_3$ | $CH_3$ | H | H | 3-Cl | H | |
| 3.53 | $CO_2C_2H_5$ | H | $CH_3$ | $CH_3$ | H | H | 1-Cl | 3-Cl | |
| 3.54 | $CO_2C_2H_5$ | H | $CH_3$ | $CH_3$ | H | H | 1-$CH_3$ | H | |
| 3.55 | $CO_2C_2H_5$ | H | $CH_3$ | $CH_3$ | H | H | 3-$CH_3$ | H | |
| 3.56 | $CO_2C_2H_5$ | H | $CH_3$ | $CH_3$ | H | H | 3-$C_4H_9$-t | H | |
| 3.57 | $CO_2C_2H_5$ | H | $-CH_2CH_2-$ | | H | H | H | H | |
| 3.58 | $CO_2C_2H_5$ | H | $-CH_2CH_2-$ | | H | H | 1-Cl | H | |
| 3.59 | $CO_2C_2H_5$ | H | $-CH_2CH_2-$ | | H | H | 3-Cl | H | |
| 3.60 | $CO_2C_2H_5$ | H | $-CH_2CH_2-$ | | H | H | 1-Cl | 3-Cl | |
| 3.61 | $CO_2C_2H_5$ | H | $C_2H_5$ | H | H | H | H | H | |
| 3.62 | $CO_2C_2H_5$ | H | $C_2H_5$ | $C_2H_5$ | H | H | H | H | |
| 3.63 | $CO_2C_2H_5$ | H | $C_2H_5$ | $C_2H_5$ | H | H | 1-Cl | H | |
| 3.64 | $CO_2C_2H_5$ | H | $C_2H_5$ | $C_2H_5$ | H | H | 3-Cl | H | |
| 3.65 | $CO_2C_2H_5$ | H | $C_2H_5$ | $C_2H_5$ | H | H | 1-Cl | 3-Cl | |
| 3.66 | $CO_2C_2H_5$ | H | $C_2H_5$ | $C_2H_5$ | H | H | 3-$CH_3$ | H | |
| 3.67 | $COCH_3$ | H | $CH_3$ | $CH_3$ | H | H | H | H | |
| 3.68 | $COCH_3$ | H | $CH_3$ | $CH_3$ | H | H | 1-Cl | H | |
| 3.69 | $COCH_3$ | H | $CH_3$ | $CH_3$ | H | H | 3-Cl | H | |
| 3.70 | $COCH_3$ | H | $CH_3$ | $CH_3$ | H | H | 1-Cl | 3-Cl | |

Tabelle 3: (Fortsetzung)

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 3.71 | $COCH_3$ | H | $CH_3$ | $CH_3$ | H | H | 1-Cl | 3-$CH_3$ | |
| 3.72 | $COC_2H_5$ | H | $-CH_2CH_2-$ | | H | H | H | H | |
| 3.73 | $CONH_2$ | H | $CH_3$ | $CH_3$ | H | H | H | H | |
| 3.74 | $CONH_2$ | H | $C_2H_5$ | H | H | H | H | H | |
| 3.75 | $CONH_2$ | H | $C_2H_5$ | $C_2H_5$ | H | H | H | H | |
| 3.76 | $CONHCH_3$ | H | $CH_3$ | $CH_3$ | H | H | H | H | |
| 3.77 | $CON(CH_3)_2$ | H | $CH_3$ | $CH_3$ | H | H | H | H | |
| 3.78 | CN | H | $CH_3$ | $CH_3$ | H | H | H | H | |
| 3.79 | $CO_2CH_2CH{=}CH_2$ | H | $CH_3$ | $CH_3$ | H | H | H | H | |
| 3.80 | $CO_2CH_2C{\equiv}CH$ | H | $CH_3$ | $CH_3$ | H | H | H | H | |
| 3.81 | $CONHOCH_3$ | H | $CH_3$ | $CH_3$ | H | H | H | H | |

Tabelle 4:

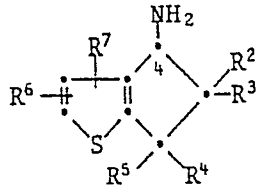

| Verb. Nr. | $R^2$ | $R^3$ | $R^4$ | $R^5$ | $R^6$ | $R^7$ | phys. Daten |
|---|---|---|---|---|---|---|---|
| 4.01 | H | H | H | H | H | H | |
| 4.02 | $CH_3$ | H | H | H | H | H | |
| 4.03 | $CH_3$ | H | H | H | H | H | 4,5-cis |
| 4.04 | $CH_3$ | H | H | H | H | H | 4,5-trans |
| 4.05 | $CH_3$ | H | $CH_3$ | H | H | H | |
| 4.06 | $CH_3$ | H | $CH_3$ | $CH_3$ | H | H | |
| 4.07 | $CH_3$ | H | H | H | 2-$CH_3$ | H | |
| 4.08 | $CH_3$ | H | H | H | 2-Cl | H | |
| 4.09 | $CH_3$ | H | H | H | 3-$CH_3$ | H | |
| 4.10 | $CH_3$ | H | H | H | 3-F | H | |
| 4.11 | $CH_3$ | H | H | H | 2-$CH_3$ | 3-Br | |
| 4.12 | $CH_3$ | H | H | H | 2-Cl | 3-Cl | |
| 4.13 | $CH_3$ | $CH_3$ | H | H | H | H | Sdp.60°C/0,08mb |
| 4.14 | $CH_3$ | $CH_3$ | $CH_3$ | H | H | H | |
| 4.15 | $CH_3$ | $CH_3$ | $CH_3$ | H | H | H | 4,6-cis |
| 4.16 | $CH_3$ | $CH_3$ | $CH_3$ | H | H | H | 4,6-trans |
| 4.17 | $CH_3$ | $CH_3$ | $CH_3$ | $CH_3$ | H | H | |
| 4.18 | $CH_3$ | $CH_3$ | H | H | 2-$CH_3$ | H | |
| 4.19 | $CH_3$ | $CH_3$ | H | H | 2-Cl | H | |
| 4.20 | $CH_3$ | $CH_3$ | H | H | 2-$C_2H_5$ | H | |
| 4.21 | $CH_3$ | $CH_3$ | H | H | 2-$C_4H_9$-t | H | |
| 4.22 | $CH_3$ | $CH_3$ | H | H | 2-CN | H | |
| 4.23 | $CH_3$ | $CH_3$ | H | H | 2-$NO_2$ | H | |
| 4.24 | $CH_3$ | $CH_3$ | H | H | 2-$CH_3$ | 3-Br | |
| 4.25 | $CH_3$ | $CH_3$ | H | H | 2-$CH_3$ | 3-$CH_3$ | |
| 4.26 | $CH_3$ | $CH_3$ | H | H | 3-$CH_3$ | H | |
| 4.27 | $CH_3$ | $CH_3$ | H | H | 3-F | H | |
| 4.28 | $CH_3$ | $CH_3$ | H | H | 3-$NO_2$ | H | |
| 4.29 | $CH_3$ | $CH_3$ | H | H | 3-Cl | H | |
| 4.30 | $-CH_2-CH_2-$ | | H | H | H | H | |
| 4.31 | $-CH_2CH_2-$ | | H | H | 2-$CH_3$ | H | |
| 4.32 | $-CH_2CH_2-$ | | H | H | 2-Cl | H | |
| 4.33 | H | H | $CH_3$ | H | H | H | |

Tabelle 4: (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 4.34 | H | H | $CH_3$ | $CH_3$ | H | H | |
| 4.35 | $C_2H_5$ | H | H | H | H | H | |
| 4.36 | $C_2H_5$ | H | H | H | H | H | 4,5-cis |
| 4.37 | $C_2H_5$ | H | H | H | H | H | 4,5-trans |
| 4.38 | $C_2H_5$ | $CH_3$ | H | H | H | H | |
| 4.39 | $C_2H_5$ | $C_2H_5$ | H | H | H | H | |
| 4.40 | $C_2H_5$ | $C_2H_5$ | H | H | 2-$CH_3$ | H | |
| 4.41 | $C_2H_5$ | $C_2H_5$ | H | H | 3-$CH_3$ | H | |
| 4.42 | $C_2H_5$ | $C_2H_5$ | H | H | 2-Cl | H | |
| 4.43 | $C_2H_5$ | $C_2H_5$ | H | H | 2-Cl | 3-$CH_3$ | |
| 4.44 | $C_2H_5$ | $C_2H_5$ | $CH_3$ | H | H | H | |
| 4.45 | $C_3H_7$-n | H | H | H | H | H | |
| 4.46 | $C_3H_7$-i | H | H | H | H | H | |
| 4.47 | $-(CH_2)_4-$ | | H | H | H | H | |
| 4.48 | $-(CH_2)_4-$ | | $CH_3$ | H | H | H | |
| 4.49 | $-(CH_2)_4-$ | | H | H | 2-Cl | H | |
| 4.50 | $-(CH_2)_4-$ | | H | H | 2-$CH_3$ | H | |
| 4.51 | $-(CH_2)_4-$ | | H | H | 3-$CH_3$ | H | |
| 4.52 | $-CH_2CH=CH_2$ | H | H | H | H | H | |
| 4.53 | $CH_2CH=CH_2$ | $CH_3$ | H | H | H | H | |
| 4.54 | $C_4H_9$-n | H | H | H | H | H | |

Tabelle 5:

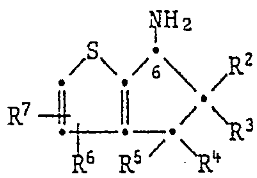

| Verb. Nr. | $R^2$ | $R^3$ | $R^4$ | $R^5$ | $R^6$ | $R^7$ | phys. Daten |
|---|---|---|---|---|---|---|---|
| 5.01 | H | H | H | H | H | H | |
| 5.02 | $CH_3$ | H | H | H | H | H | |
| 5.03 | $CH_3$ | H | H | H | H | H | 5,6-cis |
| 5.04 | $CH_3$ | H | H | H | H | H | 5,6-trans |
| 5.05 | $CH_3$ | H | $CH_3$ | H | H | H | |
| 5.06 | $CH_3$ | H | $CH_3$ | $CH_3$ | H | H | |
| 5.07 | $CH_3$ | H | H | H | $2-CH_3$ | H | |
| 5.08 | $CH_3$ | H | H | H | 2-Cl | H | |
| 5.09 | $CH_3$ | H | H | H | $2-NO_2$ | H | |
| 5.10 | $CH_3$ | H | H | H | 3-Br | $2-CH_3$ | |
| 5.11 | $CH_3$ | H | H | H | 2-Cl | 3-Cl | |
| 5.12 | $CH_3$ | $CH_3$ | H | H | H | H | |
| 5.13 | $CH_3$ | $CH_3$ | $CH_3$ | H | H | H | |
| 5.14 | $CH_3$ | $CH_3$ | $CH_3$ | H | H | H | 4,6-cis |
| 5.15 | $CH_3$ | $CH_3$ | $CH_3$ | H | H | H | 4,6-trans |
| 5.16 | $CH_3$ | $CH_3$ | $CH_3$ | $CH_3$ | H | H | |
| 5.17 | $CH_3$ | $CH_3$ | H | H | $2-CH_3$ | H | |
| 5.18 | $CH_3$ | $CH_3$ | H | H | 2-Cl | H | |
| 5.19 | $CH_3$ | $CH_3$ | H | H | $2-NO_2$ | H | |
| 5.20 | $CH_3$ | $CH_3$ | H | H | 2-CN | H | |
| 5.21 | $CH_3$ | $CH_3$ | H | H | $2-C_4H_9-t$ | H | |
| 5.22 | $CH_3$ | $CH_3$ | H | H | 3-Br | $2-CH_3$ | |
| 5.23 | $CH_3$ | $CH_3$ | H | H | 2-Cl | 3-Cl | |
| 5.24 | $CH_3$ | $CH_3$ | H | H | $2-NO_2$ | $3-CH_3$ | |
| 5.25 | $CH_3$ | $CH_3$ | H | H | $3-CH_3$ | H | |
| 5.26 | $CH_3$ | $CH_3$ | H | H | 3-CN | H | |
| 5.27 | $-CH_2CH_2-$ | | H | H | H | H | |
| 5.28 | $-CH_2CH_2-$ | | H | H | $2-CH_3$ | H | |
| 5.29 | $-CH_2CH_3-$ | | H | H | 2-Cl | H | |
| 5.30 | $-CH_2CH_2-$ | | H | H | $2-NO_2$ | H | |
| 5.31 | H | H | $CH_3$ | H | H | H | |
| 5.32 | H | H | $CH_3$ | $CH_3$ | H | H | |
| 5.33 | $C_2H_5$ | H | H | H | H | H | |
| 5.34 | $C_2H_5$ | H | H | H | H | H | 5,6-cis |
| 5.35 | $C_2H_5$ | H | H | H | H | H | 5,6-trans |

Tabelle 5 (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 5.36 | $C_2H_5$ | $CH_3$ | H | H | H | H | |
| 5.37 | $C_2H_5$ | $C_2H_5$ | H | H | H | H | |
| 5.38 | $C_2H_5$ | $C_2H_5$ | H | H | 2-$CH_3$ | H | |
| 5.39 | $C_2H_5$ | $C_2H_5$ | H | H | 2-Cl | H | |
| 5.40 | $C_2H_5$ | $C_2H_5$ | H | H | 3-$CH_3$ | 2-Cl | |
| 5.41 | $C_2H_5$ | $C_2H_5$ | $CH_3$ | H | H | H | |
| 5.42 | $C_3H_7$-n | H | H | H | H | H | |
| 5.43 | $C_3H_7$-i | H | H | H | H | H | |
| 5.44 | $-(CH_2)_4-$ | | H | H | H | H | |
| 5.45 | $-(CH_2)_4-$ | | $CH_3$ | H | H | H | |
| 5.46 | $-(CH_2)_4-$ | | H | H | 2-$CH_3$ | H | |
| 5.47 | $-(CH_2)_4-$ | | H | H | 2-Cl | H | |
| 5.48 | $-CH_2CH=CH_2$ | H | H | H | H | H | |
| 5.49 | $CH_2C\equiv CH$ | H | H | H | H | H | |
| 5.50 | $C_4H_9$-n | H | H | H | H | H | |

Tabelle 6:

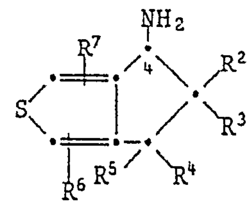

| Verb. Nr. | $R^2$ | $R^3$ | $R^4$ | $R^5$ | $R^6$ | $R^7$ | phys. Daten |
|---|---|---|---|---|---|---|---|
| 6.01 | H | H | H | H | H | H | |
| 6.02 | H | H | H | H | 1-Cl | 3-Cl | |
| 6.03 | H | H | H | H | 3-Cl | H | |
| 6.04 | H | H | H | H | 1-Cl | H | |
| 6.05 | H | H | H | H | 1-$CH_3$ | 3-$CH_3$ | |
| 6.06 | $CH_3$ | H | H | H | H | H | |
| 6.07 | $CH_3$ | H | H | H | H | H | 4,5-cis |
| 6.08 | $CH_3$ | H | H | H | H | H | 4,5-trans |
| 6.09 | $CH_3$ | H | H | H | 1-Cl | H | |
| 6.10 | $CH_3$ | H | H | H | 3-Cl | H | |
| 6.11 | $CH_3$ | H | H | H | 1-Cl | 3-Cl | |
| 6.12 | $CH_3$ | H | H | H | 1-$CH_3$ | 3-$CH_3$ | |
| 6.13 | $CH_3$ | H | $CH_3$ | H | H | H | |
| 6.14 | $CH_3$ | $CH_3$ | H | H | H | H | |
| 6.15 | $CH_3$ | $CH_3$ | H | H | 1-Cl | H | |
| 6.16 | $CH_3$ | $CH_3$ | H | H | 3-Cl | H | |
| 6.17 | $CH_3$ | $CH_3$ | H | H | 1-Cl | 3-Cl | |
| 6.18 | $CH_3$ | $CH_3$ | H | H | 1-$CH_3$ | 3-$CH_3$ | |
| 6.19 | $CH_3$ | $CH_3$ | H | H | 3-$CH_3$ | H | |
| 6.20 | $CH_3$ | $CH_3$ | H | H | 1-$NO_2$ | H | |
| 6.21 | $CH_3$ | $CH_3$ | $CH_3$ | H | H | H | |
| 6.22 | $CH_3$ | $CH_3$ | $CH_3$ | H | 1-Cl | H | |
| 6.23 | $CH_3$ | $CH_3$ | $CH_3$ | H | H | H | 4,6-cis |
| 6.24 | $CH_3$ | $CH_3$ | $CH_3$ | H | H | H | 4,6-trans |
| 6.25 | $CH_3$ | $CH_3$ | $CH_3$ | $CH_3$ | H | H | |
| 6.26 | $CH_3$ | $CH_3$ | $CH_3$ | $CH_3$ | 1-Cl | H | |
| 6.27 | $-CH_2CH_2-$ | | H | H | H | H | |
| 6.28 | $-CH_2CH_2-$ | | H | H | 1-Cl | H | |
| 6.29 | $-CH_2CH_2-$ | | H | H | 3-Cl | H | |
| 6.30 | $-CH_2CH_2-$ | | H | H | 1-Cl | 3-Cl | |
| 6.31 | H | H | $CH_3$ | H | H | H | |
| 6.32 | $C_2H_5$ | H | H | H | H | H | |
| 6.33 | $C_2H_5$ | H | H | H | H | H | 4,5-cis |
| 6.34 | $C_2H_5$ | H | H | H | H | H | 4,5-trans |

Tabelle 6 (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 6.35 | $C_2H_5$ | $C_2H_5$ | H | H | H | H | |
| 6.36 | $C_2H_5$ | $C_2H_5$ | H | H | 1-Cl | H | |
| 6.37 | $C_2H_5$ | $C_2H_5$ | H | H | 3-Cl | H | |
| 6.38 | $C_2H_5$ | $C_2H_5$ | H | H | 1-Cl | 3-Cl | |
| 6.39 | $C_3H_7$-i | H | H | H | H | H | |
| 6.40 | $-(CH_2)_4-$ | | H | H | H | H | |
| 6.41 | $-(CH_2)_4-$ | | H | H | 1-Cl | 3-Cl | |
| 6.42 | $CH_2CH{=}CH_2$ | H | H | H | H | H | |
| 6.43 | $CH_2CH{=}CH_2$ | $CH_3$ | H | H | H | H | |
| 6.44 | $C_4H_9$-n | H | H | H | H | H | |

Tabelle 7:

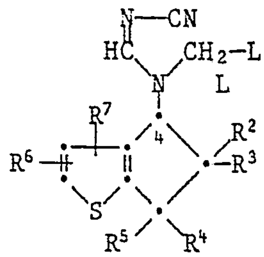

| Verb. Nr. | L | $R^2$ | $R^3$ | $R^4$ | $R^5$ | $R^6$ | $R^7$ | phys. Daten |
|---|---|---|---|---|---|---|---|---|
| 7.01 | $CO_2CH_3$ | H | H | H | H | H | H | |
| 7.02 | $CO_2CH_3$ | $CH_3$ | H | H | H | H | H | |
| 7.03 | $CO_2CH_3$ | $CH_3$ | H | H | H | H | H | 4,5-cis |
| 7.04 | $CO_2CH_3$ | $CH_3$ | H | H | H | H | H | 4,5-trans |
| 7.05 | $CO_2CH_3$ | $CH_3$ | H | $CH_3$ | H | H | H | |
| 7.06 | $CO_2CH_3$ | $CH_3$ | H | $CH_3$ | $CH_3$ | H | H | |
| 7.07 | $CO_2CH_3$ | $CH_3$ | H | H | H | 2-$CH_3$ | H | |
| 7.08 | $CO_2CH_3$ | $CH_3$ | H | H | H | 2-Cl | H | |
| 7.09 | $CO_2CH_3$ | $CH_3$ | H | H | H | 3-$CH_3$ | H | |
| 7.10 | $CO_2CH_3$ | $CH_3$ | H | H | H | 3-F | H | |
| 7.11 | $CO_2CH_3$ | $CH_3$ | H | H | H | 2-$CH_3$ | 3-Br | |
| 7.12 | $CO_2CH_3$ | $CH_3$ | H | H | H | 2-Cl | 3-Cl | |
| 7.13 | $CO_2CH_3$ | $CH_3$ | $CH_3$ | H | H | H | H | Smp.118-119°C |
| 7.14 | $CO_2CH_3$ | $CH_3$ | $CH_3$ | $CH_3$ | H | H | H | |
| 7.15 | $CO_2CH_3$ | $CH_3$ | $CH_3$ | $CH_3$ | H | H | H | 4,6-cis |
| 7.16 | $CO_2CH_3$ | $CH_3$ | $CH_3$ | $CH_3$ | H | H | H | 4,6-trans |
| 7.17 | $CO_2CH_3$ | $CH_3$ | $CH_3$ | $CH_3$ | $CH_3$ | H | H | |
| 7.18 | $CO_2CH_3$ | $CH_3$ | $CH_3$ | H | H | 2-$CH_3$ | H | |
| 7.19 | $CO_2CH_3$ | $CH_3$ | $CH_3$ | H | H | 2-Cl | H | |
| 7.20 | $CO_2CH_3$ | $CH_3$ | $CH_3$ | H | H | 2-$C_2H_5$ | H | |
| 7.21 | $CO_2CH_3$ | $CH_3$ | $CH_3$ | H | H | 2-$C_4H_9$-t | H | |
| 7.22 | $CO_2CH_3$ | $CH_3$ | $CH_3$ | H | H | 2-CN | H | |
| 7.23 | $CO_2CH_3$ | $CH_3$ | $CH_3$ | H | H | 2-$NO_2$ | H | |
| 7.24 | $CO_2CH_3$ | $CH_3$ | $CH_3$ | H | H | 2-$CH_3$ | 3-Br | |
| 7.25 | $CO_2CH_3$ | $CH_3$ | $CH_3$ | H | H | 2-$CH_3$ | 3-$CH_3$ | |
| 7.26 | $CO_2CH_3$ | $CH_3$ | $CH_3$ | H | H | 3-$CH_3$ | - | |
| 7.27 | $CO_2CH_3$ | $CH_3$ | $CH_3$ | H | H | 3-F | H | |
| 7.28 | $CO_2CH_3$ | $CH_3$ | $CH_3$ | H | H | 3-$NO_2$ | H | |
| 7.29 | $CO_2CH_3$ | $CH_3$ | $CH_3$ | H | H | 3-Cl | H | |
| 7.30 | $CO_2CH_3$ | $-CH_2-CH_2-$ | | H | H | H | H | |
| 7.31 | $CO_2CH_3$ | $-CH_2CH_2-$ | | H | H | 2-$CH_3$ | H | |
| 7.32 | $CO_2CH_3$ | $-CH_2CH_2-$ | | H | H | 2-Cl | H | |
| 7.33 | $CO_2CH_3$ | H | H | $CH_3$ | H | H | H | |

Tabelle 7: (Fortsetzung)

| Verb. Nr. | L | $R^2$ | $R^3$ | $R^4$ | $R^5$ | $R^6$ | $R^7$ | phys. Daten |
|---|---|---|---|---|---|---|---|---|
| 7.34 | $CO_2CH_3$ | H | H | $CH_3$ | $CH_3$ | H | H | |
| 7.35 | $CO_2CH_3$ | $C_2H_5$ | H | H | H | H | H | |
| 7.36 | $CO_2CH_3$ | $C_2H_5$ | H | H | H | H | H | 4,5-cis |
| 7.37 | $CO_2CH_3$ | $C_2H_5$ | H | H | H | H | H | 4,5-trans |
| 7.38 | $CO_2CH_3$ | $C_2H_5$ | $CH_3$ | H | H | H | H | |
| 7.39 | $CO_2CH_3$ | $C_2H_5$ | $C_2H_5$ | H | H | H | H | |
| 7.40 | $CO_2CH_3$ | $C_2H_5$ | $C_2H_5$ | H | H | $2-CH_3$ | H | |
| 7.41 | $CO_2CH_3$ | $C_2H_5$ | $C_2H_5$ | H | H | $3-CH_3$ | H | |
| 7.42 | $CO_2CH_3$ | $C_2H_5$ | $C_2H_5$ | H | H | 2-Cl | H | |
| 7.43 | $CO_2CH_3$ | $C_2H_5$ | $C_2H_5$ | H | H | 2-Cl | $3-CH_3$ | |
| 7.44 | $CO_2CH_3$ | $C_2H_5$ | $C_2H_5$ | $CH_3$ | H | H | H | |
| 7.45 | $CO_2CH_3$ | $C_3H_7$-n | H | H | H | H | H | |
| 7.46 | $CO_2CH_3$ | $C_3H_7$-i | H | H | H | H | H | |
| 7.47 | $CO_2CH_3$ | $-(CH_2)_4-$ | | H | H | H | H | |
| 7.48 | $CO_2CH_3$ | $-(CH_2)_4-$ | | $CH_3$ | H | H | H | |
| 7.49 | $CO_2CH_3$ | $-(CH_2)_4-$ | | H | H | 2-Cl | H | |
| 7.50 | $CO_2CH_3$ | $-(CH_2)_4-$ | | H | H | $2-CH_3$ | H | |
| 7.51 | $CO_2CH_3$ | $-(CH_2)_4-$ | | H | H | $3-CH_3$ | H | |
| 7.52 | $CO_2CH_3$ | $-CH_2CH=CH_2$ | H | H | H | H | H | |
| 7.53 | $CO_2CH_3$ | $CH_2CH=CH_2$ | $CH_3$ | H | H | H | H | |
| 7.54 | $CO_2CH_3$ | $C_4H_9$-n | H | H | H | H | H | |
| 7.55 | $CO_2C_2H_5$ | $CH_3$ | H | H | H | H | H | |
| 7.56 | $CO_2C_2H_5$ | $CH_3$ | H | $CH_3$ | H | H | H | |
| 7.57 | $CO_2C_2H_5$ | $CH_3$ | $CH_3$ | H | H | H | H | |
| 7.58 | $CO_2C_2H_5$ | $CH_3$ | $CH_3$ | $CH_3$ | H | H | H | |
| 7.59 | $CO_2C_2H_5$ | $CH_3$ | $CH_3$ | H | H | $2-CH_3$ | H | |
| 7.60 | $CO_2C_2H_5$ | $CH_3$ | $CH_3$ | H | H | 2-Cl | H | |
| 7.61 | $CO_2C_2H_5$ | $CH_3$ | $CH_3$ | H | H | 2-CN | H | |
| 7.62 | $CO_2C_2H_5$ | $CH_3$ | $CH_3$ | H | H | $2-NO_2$ | H | |
| 7.63 | $CO_2C_2H_5$ | $CH_3$ | $CH_3$ | H | H | $2-CH_3$ | 3-Cl | |
| 7.64 | $CO_2C_2H_5$ | $CH_3$ | $CH_3$ | H | H | $2-CH_3$ | $3-CH_3$ | |
| 7.65 | $CO_2C_2H_5$ | $CH_3$ | $CH_3$ | H | H | 2-Cl | $3-CH_3$ | |
| 7.66 | $CO_2C_2H_5$ | $CH_3$ | $CH_3$ | H | H | 3-F | H | |
| 7.67 | $CO_2C_2H_5$ | $-CH_2CH_2-$ | | H | H | H | H | |
| 7.68 | $CO_2C_2H_5$ | $-CH_2CH_2-$ | | H | H | 2-Cl | H | |
| 7.69 | $CO_2C_2H_5$ | $-CH_2CH_2-$ | | H | H | $2-CH_3$ | H | |
| 7.70 | $CO_2C_2H_5$ | $C_2H_5$ | H | H | H | H | H | |
| 7.71 | $CO_2C_2H_5$ | $C_2H_5$ | $C_2H_5$ | H | H | H | H | |

Tabelle 8:

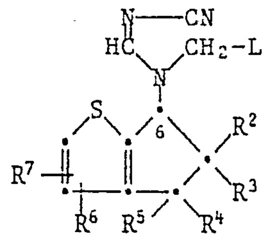

| Verb. Nr. | L | $R^2$ | $R^3$ | $R^4$ | $R^5$ | $R^6$ | $R^7$ | phys. Daten |
|---|---|---|---|---|---|---|---|---|
| 8.01 | $CO_2CH_3$ | H | H | H | H | H | H | |
| 8.02 | $CO_2CH_3$ | $CH_3$ | H | H | H | H | H | |
| 8.03 | $CO_2CH_3$ | $CH_3$ | H | H | H | H | H | 5,6-cis |
| 8.04 | $CO_2CH_3$ | $CH_3$ | H | H | H | H | H | 5,6-trans |
| 8.05 | $CO_2CH_3$ | $CH_3$ | H | $CH_3$ | H | H | H | |
| 8.06 | $CO_2CH_3$ | $CH_3$ | H | $CH_3$ | $CH_3$ | H | H | |
| 8.07 | $CO_2CH_3$ | $CH_3$ | H | H | H | $2-CH_3$ | H | |
| 8.08 | $CO_2CH_3$ | $CH_3$ | H | H | H | 2-Cl | H | |
| 8.09 | $CO_2CH_3$ | $CH_3$ | H | H | H | $2-NO_2$ | H | |
| 8.10 | $CO_2CH_3$ | $CH_3$ | H | H | H | 3-Br | $2-CH_3$ | |
| 8.11 | $CO_2CH_3$ | $CH_3$ | H | H | H | 2-Cl | 3-Cl | |
| 8.12 | $CO_2CH_3$ | $CH_3$ | $CH_3$ | H | H | H | H | |
| 8.13 | $CO_2CH_3$ | $CH_3$ | $CH_3$ | $CH_3$ | H | H | H | |
| 8.14 | $CO_2CH_3$ | $CH_3$ | $CH_3$ | $CH_3$ | H | H | H | 4,6-cis |
| 8.15 | $CO_2CH_3$ | $CH_3$ | $CH_3$ | $CH_3$ | H | H | H | 4,6-trans |
| 8.16 | $CO_2CH_3$ | $CH_3$ | $CH_3$ | $CH_3$ | $CH_3$ | H | H | |
| 8.17 | $CO_2CH_3$ | $CH_3$ | $CH_3$ | H | H | $2-CH_3$ | H | |
| 8.18 | $CO_2CH_3$ | $CH_3$ | $CH_3$ | H | H | 2-Cl | H | |
| 8.19 | $CO_2CH_3$ | $CH_3$ | $CH_3$ | H | H | $2-NO_2$ | H | |
| 8.20 | $CO_2CH_3$ | $CH_3$ | $CH_3$ | H | H | 2-CN | H | |
| 8.21 | $CO_2CH_3$ | $CH_3$ | $CH_3$ | H | H | $2-C_4H_9-t$ | H | |
| 8.22 | $CO_2CH_3$ | $CH_3$ | $CH_3$ | H | H | 3-Br | $2-CH_3$ | |
| 8.23 | $CO_2CH_3$ | $CH_3$ | $CH_3$ | H | H | 2-Cl | 3-Cl | |
| 8.24 | $CO_2CH_3$ | $CH_3$ | $CH_3$ | H | H | $2-NO_2$ | $3-CH_3$ | |
| 8.25 | $CO_2CH_3$ | $CH_3$ | $CH_3$ | H | H | $3-CH_3$ | H | |
| 8.26 | $CO_2CH_3$ | $CH_3$ | $CH_3$ | H | H | 3-CN | H | |
| 8.27 | $CO_2CH_3$ | $-CH_2CH_2-$ | | H | H | H | H | |
| 8.28 | $CO_2CH_3$ | $-CH_2CH_2-$ | | H | H | $2-CH_3$ | H | |
| 8.29 | $CO_2CH_3$ | $-CH_2CH_3-$ | | H | H | 2-Cl | H | |
| 8.30 | $CO_2CH_3$ | $-CH_2CH_2-$ | | H | H | $2-NO_2$ | H | |
| 8.31 | $CO_2CH_3$ | H | H | $CH_3$ | H | H | H | |
| 8.32 | $CO_2CH_3$ | H | H | $CH_3$ | $CH_3$ | H | H | |
| 8.33 | $CO_2CH_3$ | $C_2H_5$ | H | H | H | H | H | |
| 8.34 | $CO_2CH_3$ | $C_2H_5$ | H | H | H | H | H | 5,6-cis |
| 8.35 | $CO_2CH_3$ | $C_2H_5$ | H | H | H | H | H | 5,6-trans |

Tabelle 8 (Fortsetzung)

| Verb. Nr. | L | $R^2$ | $R^3$ | $R^4$ | $R^5$ | $R^6$ | $R^7$ | phys. Daten |
|---|---|---|---|---|---|---|---|---|
| 8.36 | $CO_2CH_3$ | $C_2H_5$ | $CH_3$ | H | H | H | H | |
| 8.37 | $CO_2CH_3$ | $C_2H_5$ | $C_2H_5$ | H | H | H | H | |
| 8.38 | $CO_2CH_3$ | $C_2H_5$ | $C_2H_5$ | H | H | 2-$CH_3$ | H | |
| 8.39 | $CO_2CH_3$ | $C_2H_5$ | $C_2H_5$ | H | H | 2-Cl | H | |
| 8.40 | $CO_2CH_3$ | $C_2H_5$ | $C_2H_5$ | H | H | 3-$CH_3$ | 2-Cl | |
| 8.41 | $CO_2CH_3$ | $C_2H_5$ | $C_2H_5$ | $CH_3$ | H | H | H | |
| 8.42 | $CO_2CH_3$ | $C_3H_7$-n | H | H | H | H | H | |
| 8.43 | $CO_2CH_3$ | $C_3H_7$-i | H | H | H | H | H | |
| 8.44 | $CO_2CH_3$ | $-(CH_2)_4-$ | | H | H | H | H | |
| 8.45 | $CO_2CH_3$ | $-(CH_2)_4-$ | | $CH_3$ | H | H | H | |
| 8.46 | $CO_2CH_3$ | $-(CH_2)_4-$ | | H | H | 2-$CH_3$ | H | |
| 8.47 | $CO_2CH_3$ | $-(CH_2)_4-$ | | H | H | 2-Cl | H | |
| 8.48 | $CO_2CH_3$ | $-CH_2CH=CH_2$ | H | H | H | H | H | |
| 8.49 | $CO_2CH_3$ | $CH_2C\equiv CH$ | H | H | H | H | H | |
| 8.50 | $CO_2CH_3$ | $C_4H_9$-n | H | H | H | H | H | |
| 8.51 | $CO_2C_2H_5$ | $CH_3$ | H | H | H | H | H | |
| 8.52 | $CO_2C_2H_5$ | $CH_3$ | H | H | H | H | H | 5,6-cis |
| 8.53 | $CO_2C_2H_5$ | $CH_3$ | H | H | H | H | H | 5,6-trans |
| 8.54 | $CO_2C_2H_5$ | $CH_3$ | $CH_3$ | H | H | H | H | |
| 8.55 | $CO_2C_2H_5$ | $CH_3$ | $CH_3$ | $CH_3$ | H | H | H | |
| 8.56 | $CO_2C_2H_5$ | $CH_3$ | $CH_3$ | $CH_3$ | $CH_3$ | H | H | |
| 8.57 | $CO_2C_2H_5$ | $CH_3$ | $CH_3$ | H | H | 2-$CH_3$ | H | |
| 8.58 | $CO_2C_2H_5$ | $CH_3$ | $CH_3$ | H | H | 2-Cl | H | |
| 8.59 | $CO_2C_2H_5$ | $CH_3$ | $CH_3$ | H | H | 2-$NO_2$ | H | |
| 8.60 | $CO_2C_2H_5$ | $CH_3$ | $CH_3$ | H | H | 2-CN | H | |
| 8.61 | $CO_2C_2H_5$ | $CH_3$ | $CH_3$ | H | H | 2-$C_4H_9$-t | H | |
| 8.62 | $CO_2C_2H_5$ | $CH_3$ | $CH_3$ | H | H | 3-Cl | 2-$CH_3$ | |
| 8.63 | $CO_2C_2H_5$ | $CH_3$ | $CH_3$ | H | H | 3-$CH_3$ | 2-Cl | |
| 8.64 | $CO_2C_2H_5$ | $CH_3$ | $CH_3$ | H | H | 3-$CH_3$ | H | |
| 8.65 | $CO_2C_2H_5$ | $CH_3$ | $CH_3$ | H | H | 2-F | H | |
| 8.66 | $CO_2C_2H_5$ | $-CH_2CH_2-$ | | H | H | H | H | |
| 8.67 | $CO_2C_2H_5$ | $-CH_2CH_2-$ | | H | H | 2-$CH_3$ | H | |
| 8.68 | $CO_2C_2H_5$ | $-CH_2CH_2-$ | | H | H | 2-Cl | H | |
| 8.69 | $CO_2C_2H_5$ | $C_2H_5$ | H | H | H | H | H | |
| 8.70 | $CO_2C_2H_5$ | $C_2H_5$ | $C_2H_5$ | H | H | H | H | |

Tabelle 9:

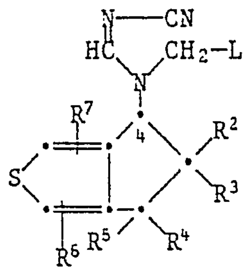

| Verb. Nr. | L | $R^2$ | $R^3$ | $R^4$ | $R^5$ | $R^6$ | $R^7$ | phys. Daten |
|---|---|---|---|---|---|---|---|---|
| 9.01 | $CO_2CH_3$ | H | H | H | H | H | H | |
| 9.02 | $CO_2CH_3$ | H | H | H | H | 1-Cl | 3-Cl | |
| 9.03 | $CO_2CH_3$ | H | H | H | H | 3-Cl | H | |
| 9.04 | $CO_2CH_3$ | H | H | H | H | 1-Cl | H | |
| 9.05 | $CO_2CH_3$ | H | H | H | H | 1-$CH_3$ | 3-$CH_3$ | |
| 9.06 | $CO_2CH_3$ | $CH_3$ | H | H | H | H | H | |
| 9.07 | $CO_2CH_3$ | $CH_3$ | H | H | H | H | H | 4,5-cis |
| 9.08 | $CO_2CH_3$ | $CH_3$ | H | H | H | H | H | 4,5-trans |
| 9.09 | $CO_2CH_3$ | $CH_3$ | H | H | H | 1-Cl | H | |
| 9.10 | $CO_2CH_3$ | $CH_3$ | H | H | H | 3-Cl | H | |
| 9.11 | $CO_2CH_3$ | $CH_3$ | H | H | H | 1-Cl | 3-Cl | |
| 9.12 | $CO_2CH_3$ | $CH_3$ | H | H | H | 1-$CH_3$ | 3-$CH_3$ | |
| 9.13 | $CO_2CH_3$ | $CH_3$ | H | $CH_3$ | H | H | H | |
| 9.14 | $CO_2CH_3$ | $CH_3$ | $CH_3$ | H | H | H | H | |
| 9.15 | $CO_2CH_3$ | $CH_3$ | $CH_3$ | H | H | 1-Cl | H | |
| 9.16 | $CO_2CH_3$ | $CH_3$ | $CH_3$ | H | H | 3-Cl | H | |
| 9.17 | $CO_2CH_3$ | $CH_3$ | $CH_3$ | H | H | 1-Cl | 3-Cl | |
| 9.18 | $CO_2CH_3$ | $CH_3$ | $CH_3$ | H | H | 1-$CH_3$ | 3-$CH_3$ | |
| 9.19 | $CO_2CH_3$ | $CH_3$ | $CH_3$ | H | H | 3-$CH_3$ | H | |
| 9.20 | $CO_2CH_3$ | $CH_3$ | $CH_3$ | H | H | 1-$NO_2$ | H | |
| 9.21 | $CO_2CH_3$ | $CH_3$ | $CH_3$ | $CH_3$ | H | H | H | |
| 9.22 | $CO_2CH_3$ | $CH_3$ | $CH_3$ | $CH_3$ | H | 1-Cl | H | |
| 9.23 | $CO_2CH_3$ | $CH_3$ | $CH_3$ | $CH_3$ | H | H | H | 4,6-cis |
| 9.24 | $CO_2CH_3$ | $CH_3$ | $CH_3$ | $CH_3$ | H | H | H | 4,6-trans |
| 9.25 | $CO_2CH_3$ | $CH_3$ | $CH_3$ | $CH_3$ | $CH_3$ | H | H | |
| 9.26 | $CO_2CH_3$ | $CH_3$ | $CH_3$ | $CH_3$ | $CH_3$ | 1-Cl | H | |
| 9.27 | $CO_2CH_3$ | $-CH_2CH_2-$ | | H | H | H | H | |
| 9.28 | $CO_2CH_3$ | $-CH_2CH_2-$ | | H | H | 1-Cl | H | |
| 9.29 | $CO_2CH_3$ | $-CH_2CH_2-$ | | H | H | 3-Cl | H | |
| 9.30 | $CO_2CH_3$ | $-CH_2CH_2-$ | | H | H | 1-Cl | 3-Cl | |
| 9.31 | $CO_2CH_3$ | H | H | $CH_3$ | H | H | H | |
| 9.32 | $CO_2CH_3$ | $C_2H_5$ | H | H | H | H | H | |
| 9.33 | $CO_2CH_3$ | $C_2H_5$ | H | H | H | H | H | 4,5-cis |
| 9.34 | $CO_2CH_3$ | $C_2H_5$ | H | H | H | H | H | 4,5-trans |

Tabelle 9 (Fortsetzung)

| Verb. Nr. | L | $R^2$ | $R^3$ | $R^4$ | $R^5$ | $R^6$ | $R^7$ | phys. Daten |
|---|---|---|---|---|---|---|---|---|
| 9.35 | $CO_2CH_3$ | $C_2H_5$ | $C_2H_5$ | H | H | H | H | |
| 9.36 | $CO_2CH_3$ | $C_2H_5$ | $C_2H_5$ | H | H | 1-Cl | H | |
| 9.37 | $CO_2CH_3$ | $C_2H_5$ | $C_2H_5$ | H | H | 3-Cl | H | |
| 9.38 | $CO_2CH_3$ | $C_2H_5$ | $C_2H_5$ | H | H | 1-Cl | 3-Cl | |
| 9.39 | $CO_2CH_3$ | $C_3H_7$-i | H | H | H | H | H | |
| 9.40 | $CO_2CH_3$ | $-(CH_2)_4-$ | | H | H | H | H | |
| 9.41 | $CO_2CH_3$ | $-(CH_2)_4-$ | | H | H | 1-Cl | 3-Cl | |
| 9.42 | $CO_2CH_3$ | $CH_2CH=CH_2$ | H | H | H | H | H | |
| 9.43 | $CO_2CH_3$ | $CH_2CH=CH_2$ | $CH_3$ | H | H | H | H | |
| 9.44 | $CO_2CH_3$ | $C_4H_9$-n | H | H | H | H | H | |
| 9.45 | $CO_2C_2H_5$ | $CH_3$ | H | H | H | 1-Cl | H | |
| 9.46 | $CO_2C_2H_5$ | $CH_3$ | H | H | H | 3-Cl | H | |
| 9.47 | $CO_2C_2H_5$ | $CH_3$ | H | H | H | 1-Cl | 3-Cl | |
| 9.48 | $CO_2C_2H_5$ | $CH_3$ | $CH_3$ | H | H | H | H | |
| 9.49 | $CO_2C_2H_5$ | $CH_3$ | $CH_3$ | H | H | 1-Cl | H | |
| 9.50 | $CO_2C_2H_5$ | $CH_3$ | $CH_3$ | H | H | 3-Cl | H | |
| 9.51 | $CO_2C_2H_5$ | $CH_3$ | $CH_3$ | H | H | 1-Cl | 3-Cl | |
| 9.52 | $CO_2C_2H_5$ | $CH_3$ | $CH_3$ | H | H | 1-$CH_3$ | H | |
| 9.53 | $CO_2C_2H_5$ | $CH_3$ | $CH_3$ | H | H | 3-$CH_3$ | H | |
| 9.54 | $CO_2C_2H_5$ | $CH_3$ | $CH_3$ | H | H | 3-$C_4H_9$-t | H | |
| 9.55 | $CO_2C_2H_5$ | $-CH_2CH_2-$ | | H | H | H | H | |
| 9.56 | $CO_2C_2H_5$ | $-CH_2CH_2-$ | | H | H | 1-Cl | H | |
| 9.57 | $CO_2C_2H_5$ | $-CH_2CH_2-$ | | H | H | 3-Cl | H | |
| 9.58 | $CO_2C_2H_5$ | $-CH_2CH_2-$ | | H | H | 1-Cl | 3-Cl | |
| 9.59 | $CO_2C_2H_5$ | $C_2H_5$ | H | H | H | H | H | |
| 9.60 | $CO_2C_2H_5$ | $C_2H_5$ | $C_2H_5$ | H | H | H | H | |
| 9.61 | $CO_2C_2H_5$ | $C_2H_5$ | $C_2H_5$ | H | H | 1-Cl | H | |
| 9.62 | $CO_2C_2H_5$ | $C_2H_5$ | $C_2H_5$ | H | H | 3-Cl | H | |
| 9.63 | $CO_2C_2H_5$ | $C_2H_5$ | $C_2H_5$ | H | H | 1-Cl | 3-Cl | |
| 9.64 | $CO_2C_2H_5$ | $C_2H_5$ | $C_2H_5$ | H | H | 3-$CH_3$ | H | |

Tabelle 10:

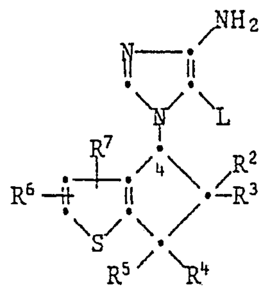

| Verb. Nr. | L | $R^2$ | $R^3$ | $R^4$ | $R^5$ | $R^6$ | $R^7$ | phys. Daten |
|---|---|---|---|---|---|---|---|---|
| 10.01 | $CO_2CH_3$ | H | H | H | H | H | H | |
| 10.02 | $CO_2CH_3$ | $CH_3$ | H | H | H | H | H | |
| 10.03 | $CO_2CH_3$ | $CH_3$ | H | H | H | H | H | 4,5-cis |
| 10.04 | $CO_2CH_3$ | $CH_3$ | H | H | H | H | H | 4,5-trans |
| 10.05 | $CO_2CH_3$ | $CH_3$ | H | $CH_3$ | H | H | H | |
| 10.06 | $CO_2CH_3$ | $CH_3$ | H | $CH_3$ | $CH_3$ | H | H | |
| 10.07 | $CO_2CH_3$ | $CH_3$ | H | H | H | 2-$CH_3$ | H | |
| 10.08 | $CO_2CH_3$ | $CH_3$ | H | H | H | 2-Cl | H | |
| 10.09 | $CO_2CH_3$ | $CH_3$ | H | H | H | 3-$CH_3$ | - | |
| 10.10 | $CO_2CH_3$ | $CH_3$ | H | H | H | 3-F | H | |
| 10.11 | $CO_2CH_3$ | $CH_3$ | H | H | H | 2-$CH_3$ | 3-Br | |
| 10.12 | $CO_2CH_3$ | $CH_3$ | H | H | H | 2-Cl | 3-Cl | |
| 10.13 | $CO_2CH_3$ | $CH_3$ | $CH_3$ | H | H | H | H | Smp.161-162°C |
| 10.14 | $CO_2CH_3$ | $CH_3$ | $CH_3$ | $CH_3$ | H | H | H | |
| 10.15 | $CO_2CH_3$ | $CH_3$ | $CH_3$ | $CH_3$ | H | H | H | 4,6-cis |
| 10.16 | $CO_2CH_3$ | $CH_3$ | $CH_3$ | $CH_3$ | H | H | H | 4,6-trans |
| 10.17 | $CO_2CH_3$ | $CH_3$ | $CH_3$ | $CH_3$ | $CH_3$ | H | H | |
| 10.18 | $CO_2CH_3$ | $CH_3$ | $CH_3$ | H | H | 2-$CH_3$ | H | |
| 10.19 | $CO_2CH_3$ | $CH_3$ | $CH_3$ | H | H | 2-Cl | H | Smp.190-192°C |
| 10.20 | $CO_2CH_3$ | $CH_3$ | $CH_3$ | H | H | 2-$C_2H_5$ | H | |
| 10.21 | $CO_2CH_3$ | $CH_3$ | $CH_3$ | H | H | 2-$C_4H_9$-t | H | |
| 10.22 | $CO_2CH_3$ | $CH_3$ | $CH_3$ | H | H | 2-CN | H | |
| 10.23 | $CO_2CH_3$ | $CH_3$ | $CH_3$ | H | H | 2-$NO_2$ | H | |
| 10.24 | $CO_2CH_3$ | $CH_3$ | $CH_3$ | H | H | 2-$CH_3$ | 3-Br | |
| 10.25 | $CO_2CH_3$ | $CH_3$ | $CH_3$ | H | H | 2-$CH_3$ | 3-$CH_3$ | |
| 10.26 | $CO_2CH_3$ | $CH_3$ | $CH_3$ | H | H | 3-$CH_3$ | H | |
| 10.27 | $CO_2CH_3$ | $CH_3$ | $CH_3$ | H | H | 3-F | - | |
| 10.28 | $CO_2CH_3$ | $CH_3$ | $CH_3$ | H | H | 3-$NO_2$ | H | |
| 10.29 | $CO_2CH_3$ | $CH_3$ | $CH_3$ | H | H | 3-Cl | H | |
| 10.30 | $CO_2CH_3$ | $-CH_2-CH_2-$ | | H | H | H | H | |
| 10.31 | $CO_2CH_3$ | $-CH_2CH_2-$ | | H | H | 2-$CH_3$ | H | |
| 10.32 | $CO_2CH_3$ | $-CH_2CH_2-$ | | H | H | 2-Cl | H | |
| 10.33 | $CO_2CH_3$ | H | H | $CH_3$ | H | H | H | |

Tabelle 10: (Fortsetzung)

| Verb. Nr. | L | $R^2$ | $R^3$ | $R^4$ | $R^5$ | $R^6$ | $R^7$ | phys. Daten |
|---|---|---|---|---|---|---|---|---|
| 10.34 | $CO_2CH_3$ | H | H | $CH_3$ | $CH_3$ | H | H | |
| 10.35 | $CO_2CH_3$ | $C_2H_5$ | H | H | H | H | H | |
| 10.36 | $CO_2CH_3$ | $C_2H_5$ | H | H | H | H | H | 4,5-cis |
| 10.37 | $CO_2CH_3$ | $C_2H_5$ | H | H | H | H | H | 4,5-trans |
| 10.38 | $CO_2CH_3$ | $C_2H_5$ | $CH_3$ | H | H | H | H | |
| 10.39 | $CO_2CH_3$ | $C_2H_5$ | $C_2H_5$ | H | H | H | H | |
| 10.40 | $CO_2CH_3$ | $C_2H_5$ | $C_2H_5$ | H | H | 2-$CH_3$ | H | |
| 10.41 | $CO_2CH_3$ | $C_2H_5$ | $C_2H_5$ | H | H | 3-$CH_3$ | H | |
| 10.42 | $CO_2CH_3$ | $C_2H_5$ | $C_2H_5$ | H | H | 2-Cl | H | |
| 10.43 | $CO_2CH_3$ | $C_2H_5$ | $C_2H_5$ | H | H | 2-Cl | 3-$CH_3$ | |
| 10.44 | $CO_2CH_3$ | $C_2H_5$ | $C_2H_5$ | $CH_3$ | H | H | H | |
| 10.45 | $CO_2CH_3$ | $C_3H_7$-n | H | H | H | H | H | |
| 10.46 | $CO_2CH_3$ | $C_3H_7$-i | H | H | H | H | H | |
| 10.47 | $CO_2CH_3$ | $-(CH_2)_4-$ | | H | H | H | H | |
| 10.48 | $CO_2CH_3$ | $-(CH_2)_4-$ | | $CH_3$ | H | H | H | |
| 10.49 | $CO_2CH_3$ | $-(CH_2)_4-$ | | H | H | 2-Cl | H | |
| 10.50 | $CO_2CH_3$ | $-(CH_2)_4-$ | | H | H | 2-$CH_3$ | H | |
| 10.51 | $CO_2CH_3$ | $-(CH_2)_4-$ | | H | H | 3-$CH_3$ | H | |
| 10.52 | $CO_2CH_3$ | $-CH_2CH=CH_2$ | H | H | H | H | H | |
| 10.53 | $CO_2CH_3$ | $CH_2CH=CH_2$ | $CH_3$ | H | H | H | H | |
| 10.54 | $CO_2CH_3$ | $C_4H_9$-n | H | H | H | H | H | |
| 10.55 | $CO_2C_2H_5$ | $CH_3$ | H | H | H | H | H | |
| 10.56 | $CO_2C_2H_5$ | $CH_3$ | H | $CH_3$ | H | H | H | |
| 10.57 | $CO_2C_2H_5$ | $CH_3$ | $CH_3$ | H | H | H | H | |
| 10.58 | $CO_2C_2H_5$ | $CH_3$ | $CH_3$ | $CH_3$ | H | H | H | |
| 10.59 | $CO_2C_2H_5$ | $CH_3$ | $CH_3$ | H | H | 2-$CH_3$ | H | |
| 10.60 | $CO_2C_2H_5$ | $CH_3$ | $CH_3$ | H | H | 2-Cl | H | |
| 10.61 | $CO_2C_2H_5$ | $CH_3$ | $CH_3$ | H | H | 2-CN | H | |
| 10.62 | $CO_2C_2H_5$ | $CH_3$ | $CH_3$ | H | H | 2-$NO_2$ | H | |
| 10.63 | $CO_2C_2H_5$ | $CH_3$ | $CH_3$ | H | H | 2-$CH_3$ | 3-Cl | |
| 10.64 | $CO_2C_2H_5$ | $CH_3$ | $CH_3$ | H | H | 2-$CH_3$ | 3-$CH_3$ | |
| 10.65 | $CO_2C_2H_5$ | $CH_3$ | $CH_3$ | H | H | 2-Cl | 3-$CH_3$ | |
| 10.66 | $CO_2C_2H_5$ | $CH_3$ | $CH_3$ | H | H | 3-F | H | |
| 10.67 | $CO_2C_2H_5$ | $-CH_2CH_2-$ | | H | H | H | H | |
| 10.68 | $CO_2C_2H_5$ | $-CH_2CH_2-$ | | H | H | 2-Cl | H | |
| 10.69 | $CO_2C_2H_5$ | $-CH_2CH_2-$ | | H | H | 2-$CH_3$ | H | |
| 10.70 | $CO_2C_2H_5$ | $C_2H_5$ | H | H | H | H | H | |
| 10.71 | $CO_2C_2H_5$ | $C_2H_5$ | $C_2H_5$ | H | H | H | H | |

Tabelle 11:

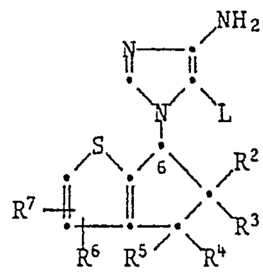

| Verb. Nr. | L | $R^2$ | $R^3$ | $R^4$ | $R^5$ | $R^6$ | $R^7$ | phys. Daten |
|---|---|---|---|---|---|---|---|---|
| 11.01 | $CO_2CH_3$ | H | H | H | H | H | H | |
| 11.02 | $CO_2CH_3$ | $CH_3$ | H | H | H | H | H | |
| 11.03 | $CO_2CH_3$ | $CH_3$ | H | H | H | H | H | 5,6-cis |
| 11.04 | $CO_2CH_3$ | $CH_3$ | H | H | H | H | H | 5,6-trans |
| 11.05 | $CO_2CH_3$ | $CH_3$ | H | $CH_3$ | H | H | H | |
| 11.06 | $CO_2CH_3$ | $CH_3$ | H | $CH_3$ | $CH_3$ | H | H | |
| 11.07 | $CO_2CH_3$ | $CH_3$ | H | H | H | 2-$CH_3$ | H | |
| 11.08 | $CO_2CH_3$ | $CH_3$ | H | H | H | 2-Cl | H | |
| 11.09 | $CO_2CH_3$ | $CH_3$ | H | H | H | 2-$NO_2$ | H | |
| 11.10 | $CO_2CH_3$ | $CH_3$ | H | H | H | 3-Br | 2-$CH_3$ | |
| 11.11 | $CO_2CH_3$ | $CH_3$ | H | H | H | 2-Cl | 3-Cl | |
| 11.12 | $CO_2CH_3$ | $CH_3$ | $CH_3$ | H | H | H | H | |
| 11.13 | $CO_2CH_3$ | $CH_3$ | $CH_3$ | $CH_3$ | H | H | H | |
| 11.14 | $CO_2CH_3$ | $CH_3$ | $CH_3$ | $CH_3$ | H | H | H | 4,6-cis |
| 11.15 | $CO_2CH_3$ | $CH_3$ | $CH_3$ | $CH_3$ | H | H | H | 4,6-trans |
| 11.16 | $CO_2CH_3$ | $CH_3$ | $CH_3$ | $CH_3$ | $CH_3$ | H | H | |
| 11.17 | $CO_2CH_3$ | $CH_3$ | $CH_3$ | H | H | 2-$CH_3$ | H | |
| 11.18 | $CO_2CH_3$ | $CH_3$ | $CH_3$ | H | H | 2-Cl | H | |
| 11.19 | $CO_2CH_3$ | $CH_3$ | $CH_3$ | H | H | 2-$NO_2$ | H | |
| 11.20 | $CO_2CH_3$ | $CH_3$ | $CH_3$ | H | H | 2-CN | H | |
| 11.21 | $CO_2CH_3$ | $CH_3$ | $CH_3$ | H | H | 2-$C_4H_9$-t | H | |
| 11.22 | $CO_2CH_3$ | $CH_3$ | $CH_3$ | H | H | 3-Br | 2-$CH_3$ | |
| 11.23 | $CO_2CH_3$ | $CH_3$ | $CH_3$ | H | H | 2-Cl | 3-Cl | |
| 11.24 | $CO_2CH_3$ | $CH_3$ | $CH_3$ | H | H | 2-$NO_2$ | 3-$CH_3$ | |
| 11.25 | $CO_2CH_3$ | $CH_3$ | $CH_3$ | H | H | 3-$CH_3$ | H | |
| 11.26 | $CO_2CH_3$ | $CH_3$ | $CH_3$ | H | H | 3-CN | H | |
| 11.27 | $CO_2CH_3$ | $-CH_2CH_2-$ | | H | H | H | H | |
| 11.28 | $CO_2CH_3$ | $-CH_2CH_2-$ | | H | H | 2-$CH_3$ | H | |
| 11.29 | $CO_2CH_3$ | $-CH_2CH_3-$ | | H | H | 2-Cl | H | |
| 11.30 | $CO_2CH_3$ | $-CH_2CH_2-$ | | H | H | 2-$NO_2$ | H | |
| 11.31 | $CO_2CH_3$ | H | H | $CH_3$ | H | H | H | |
| 11.32 | $CO_2CH_3$ | H | H | $CH_3$ | $CH_3$ | H | H | |
| 11.33 | $CO_2CH_3$ | $C_2H_5$ | H | H | H | H | H | |
| 11.34 | $CO_2CH_3$ | $C_2H_5$ | H | H | H | H | H | 5,6-cis |
| 11.35 | $CO_2CH_3$ | $C_2H_5$ | H | H | H | H | H | 5,6-trans |

Tabelle 11 (Fortsetzung)

| Verb. Nr. | L | $R^2$ | $R^3$ | $R^4$ | $R^5$ | $R^6$ | $R^7$ | phys. Daten |
|---|---|---|---|---|---|---|---|---|
| 11.36 | $CO_2CH_3$ | $C_2H_5$ | $CH_3$ | H | H | H | H | |
| 11.37 | $CO_2CH_3$ | $C_2H_5$ | $C_2H_5$ | H | H | H | H | |
| 11.38 | $CO_2CH_3$ | $C_2H_5$ | $C_2H_5$ | H | H | 2-$CH_3$ | H | |
| 11.39 | $CO_2CH_3$ | $C_2H_5$ | $C_2H_5$ | H | H | 2-Cl | H | |
| 11.40 | $CO_2CH_3$ | $C_2H_5$ | $C_2H_5$ | H | H | 3-$CH_3$ | 2-Cl | |
| 11.41 | $CO_2CH_3$ | $C_2H_5$ | $C_2H_5$ | $CH_3$ | H | H | H | |
| 11.42 | $CO_2CH_3$ | $C_3H_7$-n | H | H | H | H | H | |
| 11.43 | $CO_2CH_3$ | $C_3H_7$-i | H | H | H | H | H | |
| 11.44 | $CO_2CH_3$ | $-(CH_2)_4-$ | | H | H | H | H | |
| 11.45 | $CO_2CH_3$ | $-(CH_2)_4-$ | | $CH_3$ | H | H | H | |
| 11.46 | $CO_2CH_3$ | $-(CH_2)_4-$ | | H | H | 2-$CH_3$ | H | |
| 11.47 | $CO_2CH_3$ | $-(CH_2)_4-$ | | H | H | 2-Cl | H | |
| 11.48 | $CO_2CH_3$ | $-CH_2CH=CH_2$ | H | H | H | H | H | |
| 11.49 | $CO_2CH_3$ | $CH_2C\equiv CH$ | H | H | H | H | H | |
| 11.50 | $CO_2CH_3$ | $C_4H_9$-n | H | H | H | H | H | |
| 11.51 | $CO_2C_2H_5$ | $CH_3$ | H | H | H | H | H | |
| 11.52 | $CO_2C_2H_5$ | $CH_3$ | H | H | H | H | H | 5,6-cis |
| 11.53 | $CO_2C_2H_5$ | $CH_3$ | H | H | H | H | H | 5,6-trans |
| 11.54 | $CO_2C_2H_5$ | $CH_3$ | $CH_3$ | H | H | H | H | |
| 11.55 | $CO_2C_2H_5$ | $CH_3$ | $CH_3$ | $CH_3$ | H | H | H | |
| 11.56 | $CO_2C_2H_5$ | $CH_3$ | $CH_3$ | $CH_3$ | $CH_3$ | H | H | |
| 11.57 | $CO_2C_2H_5$ | $CH_3$ | $CH_3$ | H | H | 2-$CH_3$ | H | |
| 11.58 | $CO_2C_2H_5$ | $CH_3$ | $CH_3$ | H | H | 2-Cl | H | |
| 11.59 | $CO_2C_2H_5$ | $CH_3$ | $CH_3$ | H | H | 2-$NO_2$ | H | |
| 11.60 | $CO_2C_2H_5$ | $CH_3$ | $CH_3$ | H | H | 2-CN | H | |
| 11.61 | $CO_2C_2H_5$ | $CH_3$ | $CH_3$ | H | H | 2-$C_4H_9$-t | H | |
| 11.62 | $CO_2C_2H_5$ | $CH_3$ | $CH_3$ | H | H | 3-Cl | 2-$CH_3$ | |
| 11.63 | $CO_2C_2H_5$ | $CH_3$ | $CH_3$ | H | H | 3-$CH_3$ | 2-Cl | |
| 11.64 | $CO_2C_2H_5$ | $CH_3$ | $CH_3$ | H | H | 3-$CH_3$ | H | |
| 11.65 | $CO_2C_2H_5$ | $CH_3$ | $CH_3$ | H | H | 2-F | H | |
| 11.66 | $CO_2C_2H_5$ | $-CH_2CH_2-$ | | H | H | H | H | |
| 11.67 | $CO_2C_2H_5$ | $-CH_2CH_2-$ | | H | H | 2-$CH_3$ | H | |
| 11.68 | $CO_2C_2H_5$ | $-CH_2CH_2-$ | | H | H | 2-Cl | H | |
| 11.69 | $CO_2C_2H_5$ | $C_2H_5$ | H | H | H | H | H | |
| 11.70 | $CO_2C_2H_5$ | $C_2H_5$ | $C_2H_5$ | H | H | H | H | |

Tabelle 12:

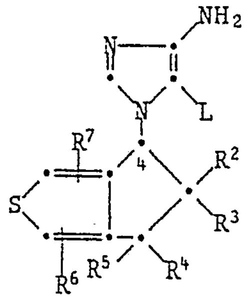

| Verb. Nr. | L | $R^2$ | $R^3$ | $R^4$ | $R^5$ | $R^6$ | $R^7$ | phys. Daten |
|---|---|---|---|---|---|---|---|---|
| 12.01 | $CO_2CH_3$ | H | H | H | H | H | H | |
| 12.02 | $CO_2CH_3$ | H | H | H | H | 1-Cl | 3-Cl | |
| 12.03 | $CO_2CH_3$ | H | H | H | H | 3-Cl | H | |
| 12.04 | $CO_2CH_3$ | H | H | H | H | 1-Cl | H | |
| 12.05 | $CO_2CH_3$ | H | H | H | H | 1-$CH_3$ | 3-$CH_3$ | |
| 12.06 | $CO_2CH_3$ | $CH_3$ | H | H | H | H | H | |
| 12.07 | $CO_2CH_3$ | $CH_3$ | H | H | H | H | H | 4,5-cis |
| 12.08 | $CO_2CH_3$ | $CH_3$ | H | H | H | H | H | 4,5-trans |
| 12.09 | $CO_2CH_3$ | $CH_3$ | H | H | H | 1-Cl | H | |
| 12.10 | $CO_2CH_3$ | $CH_3$ | H | H | H | 3-Cl | H | |
| 12.11 | $CO_2CH_3$ | $CH_3$ | H | H | H | 1-Cl | 3-Cl | |
| 12.12 | $CO_2CH_3$ | $CH_3$ | H | H | H | 1-$CH_3$ | 3-$CH_3$ | |
| 12.13 | $CO_2CH_3$ | $CH_3$ | H | $CH_3$ | H | H | H | |
| 12.14 | $CO_2CH_3$ | $CH_3$ | $CH_3$ | H | H | H | H | |
| 12.15 | $CO_2CH_3$ | $CH_3$ | $CH_3$ | H | H | 1-Cl | H | |
| 12.16 | $CO_2CH_3$ | $CH_3$ | $CH_3$ | H | H | 3-Cl | H | |
| 12.17 | $CO_2CH_3$ | $CH_3$ | $CH_3$ | H | H | 1-Cl | 3-Cl | |
| 12.18 | $CO_2CH_3$ | $CH_3$ | $CH_3$ | H | H | 1-$CH_3$ | 3-$CH_3$ | |
| 12.19 | $CO_2CH_3$ | $CH_3$ | $CH_3$ | H | H | 3-$CH_3$ | H | |
| 12.20 | $CO_2CH_3$ | $CH_3$ | $CH_3$ | H | H | 1-$NO_2$ | H | |
| 12.21 | $CO_2CH_3$ | $CH_3$ | $CH_3$ | $CH_3$ | H | H | H | |
| 12.22 | $CO_2CH_3$ | $CH_3$ | $CH_3$ | $CH_3$ | H | 1-Cl | H | |
| 12.23 | $CO_2CH_3$ | $CH_3$ | $CH_3$ | $CH_3$ | H | H | H | 4,6-cis |
| 12.24 | $CO_2CH_3$ | $CH_3$ | $CH_3$ | $CH_3$ | H | H | H | 4,6-trans |
| 12.25 | $CO_2CH_3$ | $CH_3$ | $CH_3$ | $CH_3$ | $CH_3$ | H | H | |
| 12.26 | $CO_2CH_3$ | $CH_3$ | $CH_3$ | $CH_3$ | $CH_3$ | 1-Cl | H | |
| 12.27 | $CO_2CH_3$ | $-CH_2CH_2-$ | | H | H | H | H | |
| 12.28 | $CO_2CH_3$ | $-CH_2CH_2-$ | | H | H | 1-Cl | H | |
| 12.29 | $CO_2CH_3$ | $-CH_2CH_2-$ | | H | H | 3-Cl | H | |
| 12.30 | $CO_2CH_3$ | $-CH_2CH_2-$ | | H | H | 1-Cl | 3-Cl | |
| 12.31 | $CO_2CH_3$ | H | H | $CH_3$ | H | H | H | |
| 12.32 | $CO_2CH_3$ | $C_2H_5$ | H | H | H | H | H | |
| 12.33 | $CO_2CH_3$ | $C_2H_5$ | H | H | H | H | H | 4,5-cis |
| 12.34 | $CO_2CH_3$ | $C_2H_5$ | H | H | H | H | H | 4,5-trans |

Tabelle 12 (Fortsetzung)

| Verb. Nr. | L | $R^2$ | $R^3$ | $R^4$ | $R^5$ | $R^6$ | $R^7$ | phys. Daten |
|---|---|---|---|---|---|---|---|---|
| 12.35 | $CO_2CH_3$ | $C_2H_5$ | $C_2H_5$ | H | H | H | H | |
| 12.36 | $CO_2CH_3$ | $C_2H_5$ | $C_2H_5$ | H | H | 1-Cl | H | |
| 12.37 | $CO_2CH_3$ | $C_2H_5$ | $C_2H_5$ | H | H | 3-Cl | H | |
| 12.38 | $CO_2CH_3$ | $C_2H_5$ | $C_2H_5$ | H | H | 1-Cl | 3-Cl | |
| 12.39 | $CO_2CH_3$ | $C_3H_7$-i | H | H | H | H | H | |
| 12.40 | $CO_2CH_3$ | $-(CH_2)_4-$ | | H | H | H | H | |
| 12.41 | $CO_2CH_3$ | $-(CH_2)_4-$ | | H | H | 1-Cl | 3-Cl | |
| 12.42 | $CO_2CH_3$ | $CH_2CH=CH_2$ | H | H | H | H | H | |
| 12.43 | $CO_2CH_3$ | $CH_2CH=CH_2$ | $CH_3$ | H | H | H | H | |
| 12.44 | $CO_2CH_3$ | $C_4H_9$-n | H | H | H | H | H | |
| 12.45 | $CO_2C_2H_5$ | $CH_3$ | H | H | H | 1-Cl | H | |
| 12.46 | $CO_2C_2H_5$ | $CH_3$ | H | H | H | 3-Cl | H | |
| 12.47 | $CO_2C_2H_5$ | $CH_3$ | H | H | H | 1-Cl | 3-Cl | |
| 12.48 | $CO_2C_2H_5$ | $CH_3$ | $CH_3$ | H | H | H | H | |
| 12.49 | $CO_2C_2H_5$ | $CH_3$ | $CH_3$ | H | H | 1-Cl | H | |
| 12.50 | $CO_2C_2H_5$ | $CH_3$ | $CH_3$ | H | H | 3-Cl | H | |
| 12.51 | $CO_2C_2H_5$ | $CH_3$ | $CH_3$ | H | H | 1-Cl | 3-Cl | |
| 12.52 | $CO_2C_2H_5$ | $CH_3$ | $CH_3$ | H | H | $1-CH_3$ | H | |
| 12.53 | $CO_2C_2H_5$ | $CH_3$ | $CH_3$ | H | H | $3-CH_3$ | H | |
| 12.54 | $CO_2C_2H_5$ | $CH_3$ | $CH_3$ | H | H | $3-C_4H_9$-t | H | |
| 12.55 | $CO_2C_2H_5$ | $-CH_2CH_2-$ | | H | H | H | H | |
| 12.56 | $CO_2C_2H_5$ | $-CH_2CH_2-$ | | H | H | 1-Cl | H | |
| 12.57 | $CO_2C_2H_5$ | $-CH_2CH_2-$ | | H | H | 3-Cl | H | |
| 12.58 | $CO_2C_2H_5$ | $-CH_2CH_2-$ | | H | H | 1-Cl | 3-Cl | |
| 12.59 | $CO_2C_2H_5$ | $C_2H_5$ | H | H | H | H | H | |
| 12.60 | $CO_2C_2H_5$ | $C_2H_5$ | $C_2H_5$ | H | H | H | H | |
| 12.61 | $CO_2C_2H_5$ | $C_2H_5$ | $C_2H_5$ | H | H | 1-Cl | H | |
| 12.62 | $CO_2C_2H_5$ | $C_2H_5$ | $C_2H_5$ | H | H | 3-Cl | H | |
| 12.63 | $CO_2C_2H_5$ | $C_2H_5$ | $C_2H_5$ | H | H | 1-Cl | 3-Cl | |
| 12.64 | $CO_2C_2H_5$ | $C_2H_5$ | $C_2H_5$ | H | H | $3-CH_3$ | H | |

Tabelle 13:

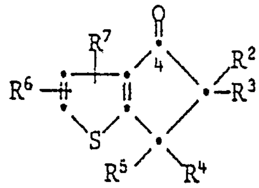

| Verb. Nr. | $R^2$ | $R^3$ | $R^4$ | $R^5$ | $R^6$ | $R^7$ | phys. Daten |
|---|---|---|---|---|---|---|---|
| 13.01 | H | H | H | H | H | H | |
| 13.02 | $CH_3$ | H | H | H | H | H | Sdp.64°C/0,1 mb |
| 13.03 | $CH_3$ | H | $CH_3$ | H | H | H | |
| 13.04 | $CH_3$ | H | $CH_3$ | $CH_3$ | H | H | |
| 13.05 | $CH_3$ | H | H | H | 2-$CH_3$ | H | |
| 13.06 | $CH_3$ | H | H | H | 2-Cl | H | |
| 13.07 | $CH_3$ | H | H | H | 3-$CH_3$ | - | |
| 13.08 | $CH_3$ | H | H | H | 3-F | H | |
| 13.09 | $CH_3$ | H | H | H | 2-$CH_3$ | 3-Br | |
| 13.10 | $CH_3$ | H | H | H | 2-Cl | 3-Cl | |
| 13.11 | $CH_3$ | $CH_3$ | H | H | H | H | Sdp.43°C/0.03mb |
| 13.12 | $CH_3$ | $CH_3$ | $CH_3$ | H | H | H | |
| 13.13 | $CH_3$ | $CH_3$ | $CH_3$ | $CH_3$ | H | H | |
| 13.14 | $CH_3$ | $CH_3$ | H | H | 2-$CH_3$ | H | |
| 13.15 | $CH_3$ | $CH_3$ | H | H | 2-Cl | H | Sdp.80-95°C/0,05 mb |
| 13.16 | $CH_3$ | $CH_3$ | H | H | 2-$C_2H_5$ | H | |
| 13.17 | $CH_3$ | $CH_3$ | H | H | 2-$C_4H_9$-t | H | |
| 13.18 | $CH_3$ | $CH_3$ | H | H | 2-CN | H | |
| 13.19 | $CH_3$ | $CH_3$ | H | H | 2-$NO_2$ | H | |
| 13.20 | $CH_3$ | $CH_3$ | H | H | 2-$CH_3$ | 3-Br | |
| 13.21 | $CH_3$ | $CH_3$ | H | H | 2-$CH_3$ | 3-$CH_3$ | |
| 13.22 | $CH_3$ | $CH_3$ | H | H | 3-$CH_3$ | H | |
| 13.23 | $CH_3$ | $CH_3$ | H | H | 3-F | H | |
| 13.24 | $CH_3$ | $CH_3$ | H | H | 3-$NO_2$ | H | |
| 13.25 | $CH_3$ | $CH_3$ | H | H | 3-Cl | H | |
| 13.26 | $-CH_2-CH_2-$ | | H | H | H | H | |
| 13.27 | $-CH_2CH_2-$ | | H | H | 2-$CH_3$ | H | |
| 13.28 | $-CH_2CH_2-$ | | H | H | 2-Cl | H | |
| 13.29 | H | H | $CH_3$ | H | H | H | |

Tabelle 13: (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 13.30 | H | H | $CH_3$ | $CH_3$ | H | H | |
| 13.31 | $C_2H_5$ | H | H | H | H | H | |
| 13.32 | $C_2H_5$ | $CH_3$ | H | H | H | H | |
| 13.33 | $C_2H_5$ | $C_2H_5$ | H | H | H | H | |
| 13.34 | $C_2H_5$ | $C_2H_5$ | H | H | 2-$CH_3$ | H | |
| 13.35 | $C_2H_5$ | $C_2H_5$ | H | H | 3-$CH_3$ | - | |
| 13.36 | $C_2H_5$ | $C_2H_5$ | H | H | 2-Cl | H | |
| 13.37 | $C_2H_5$ | $C_2H_5$ | H | H | 2-Cl | 3-$CH_3$ | |
| 13.38 | $C_2H_5$ | $C_2H_5$ | $CH_3$ | H | H | H | |
| 13.39 | $C_3H_7$-n | H | H | H | H | H | |
| 13.40 | $C_3H_7$-i | H | H | H | H | H | |
| 13.41 | $-(CH_2)_4-$ | | H | H | H | H | |
| 13.42 | $-(CH_2)_4-$ | | $CH_3$ | H | H | H | |
| 13.43 | $-(CH_2)_4-$ | | H | H | 2-Cl | H | |
| 13.44 | $-(CH_2)_4-$ | | H | H | 2-$CH_3$ | H | |
| 13.45 | $-(CH_2)_4-$ | | H | H | 3-$CH_3$ | H | |
| 13.46 | $-CH_2CH=CH_2$ | H | H | H | H | H | |
| 13.47 | $CH_2CH=CH_2$ | $CH_3$ | H | H | H | H | |
| 13.48 | $C_4H_9$-n | H | H | H | H | H | |

Tabelle 14:

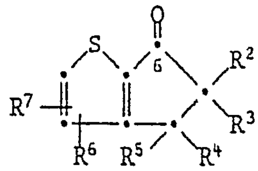

| Verb. Nr. | $R^2$ | $R^3$ | $R^4$ | $R^5$ | $R^6$ | $R^7$ | phys. Daten |
|---|---|---|---|---|---|---|---|
| 14.01 | H | H | H | H | H | H | |
| 14.02 | $CH_3$ | H | H | H | H | H | Sdp.123°C/16 mb |
| 14.03 | $CH_3$ | H | $CH_3$ | H | H | H | |
| 14.04 | $CH_3$ | H | $CH_3$ | $CH_3$ | H | H | |
| 14.05 | $CH_3$ | H | H | H | 2-$CH_3$ | H | |
| 14.06 | $CH_3$ | H | H | H | 2-Cl | H | |
| 14.07 | $CH_3$ | H | H | H | 2-$NO_2$ | H | |
| 14.08 | $CH_3$ | H | H | H | 3-Br | 2-$CH_3$ | |
| 14.09 | $CH_3$ | H | H | H | 2-Cl | 3-Cl | |
| 14.10 | $CH_3$ | $CH_3$ | H | H | H | H | |
| 14.11 | $CH_3$ | $CH_3$ | $CH_3$ | H | H | H | |
| 14.12 | $CH_3$ | $CH_3$ | $CH_3$ | $CH_3$ | H | H | |
| 14.13 | $CH_3$ | $CH_3$ | H | H | 2-$CH_3$ | H | |
| 14.14 | $CH_3$ | $CH_3$ | H | H | 2-Cl | H | |
| 14.15 | $CH_3$ | $CH_3$ | H | H | 2-$NO_2$ | H | |
| 14.16 | $CH_3$ | $CH_3$ | H | H | 2-CN | H | |
| 14.17 | $CH_3$ | $CH_3$ | H | H | 2-$C_4H_9$-t | H | |
| 14.18 | $CH_3$ | $CH_3$ | H | H | 3-Br | 2-$CH_3$ | |
| 14.19 | $CH_3$ | $CH_3$ | H | H | 2-Cl | 3-Cl | |
| 14.20 | $CH_3$ | $CH_3$ | H | H | 2-$NO_2$ | 3-$CH_3$ | |
| 14.21 | $CH_3$ | $CH_3$ | H | H | 3-$CH_3$ | H | |
| 14.22 | $CH_3$ | $CH_3$ | H | H | 3-CN | H | |
| 14.23 | $-CH_2CH_2-$ | | H | H | H | H | |
| 14.24 | $-CH_2CH_2-$ | | H | H | 2-$CH_3$ | H | |
| 14.25 | $-CH_2CH_3-$ | | H | H | 2-Cl | H | |
| 14.26 | $-CH_2CH_2-$ | | H | H | 2-$NO_2$ | H | |
| 14.27 | H | H | $CH_3$ | H | H | H | |
| 14.28 | H | H | $CH_3$ | $CH_3$ | H | H | |
| 14.29 | $C_2H_5$ | H | H | H | H | H | |

Tabelle 14 (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 14.30 | $C_2H_5$ | $CH_3$ | H | H | H | H | |
| 14.31 | $C_2H_5$ | $C_2H_5$ | H | H | H | H | |
| 14.32 | $C_2H_5$ | $C_2H_5$ | H | H | 2-$CH_3$ | H | |
| 14.33 | $C_2H_5$ | $C_2H_5$ | H | H | 2-Cl | H | |
| 14.34 | $C_2H_5$ | $C_2H_5$ | H | H | 3-$CH_3$ | 2-Cl | |
| 14.35 | $C_2H_5$ | $C_2H_5$ | $CH_3$ | H | H | H | |
| 14.36 | $C_3H_7$-n | H | H | H | H | H | |
| 14.37 | $C_3H_7$-i | H | H | H | H | H | |
| 14.38 | $-(CH_2)_4-$ | | H | H | H | H | |
| 14.39 | $-(CH_2)_4-$ | | $CH_3$ | H | H | H | |
| 14.40 | $-(CH_2)_4-$ | | H | H | 2-$CH_3$ | H | |
| 14.41 | $-(CH_2)_4-$ | | H | H | 2-Cl | H | |
| 14.42 | $-CH_2CH=CH_2$ | H | H | H | H | H | |
| 14.43 | $CH_2C{\equiv}CH$ | H | H | H | H | H | |
| 14.44 | $C_4H_9$-n | H | H | H | H | H | |

Tabelle 15:

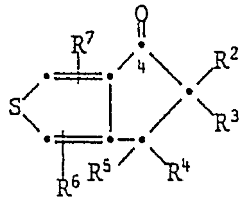

| Verb. Nr. | $R^2$ | $R^3$ | $R^4$ | $R^5$ | $R^6$ | $R^7$ | phys. Daten |
|---|---|---|---|---|---|---|---|
| 15.01 | H | H | H | H | H | H | |
| 15.02 | H | H | H | H | 1-Cl | 3-Cl | |
| 15.03 | H | H | H | H | 3-Cl | H | |
| 15.04 | H | H | H | H | 1-Cl | H | |
| 15.05 | H | H | H | H | $1-CH_3$ | $3-CH_3$ | |
| 15.06 | $CH_3$ | H | H | H | H | H | |
| 15.07 | $CH_3$ | H | H | H | 1-Cl | H | |
| 15.08 | $CH_3$ | H | H | H | 3-Cl | H | |
| 15.09 | $CH_3$ | H | H | H | 1-Cl | 3-Cl | |
| 15.10 | $CH_3$ | H | H | H | $1-CH_3$ | $3-CH_3$ | |
| 15.11 | $CH_3$ | H | $CH_3$ | H | H | H | |
| 15.12 | $CH_3$ | $CH_3$ | H | H | H | H | |
| 15.13 | $CH_3$ | $CH_3$ | H | H | 1-Cl | H | |
| 15.14 | $CH_3$ | $CH_3$ | H | H | 3-Cl | H | |
| 15.15 | $CH_3$ | $CH_3$ | H | H | 1-Cl | 3-Cl | |
| 15.16 | $CH_3$ | $CH_3$ | H | H | $1-CH_3$ | $3-CH_3$ | |
| 15.17 | $CH_3$ | $CH_3$ | H | H | $3-CH_3$ | H | |
| 15.18 | $CH_3$ | $CH_3$ | H | H | $1-NO_2$ | H | |
| 15.19 | $CH_3$ | $CH_3$ | $CH_3$ | H | H | H | |
| 15.20 | $CH_3$ | $CH_3$ | $CH_3$ | H | 1-Cl | H | |
| 15.21 | $CH_3$ | $CH_3$ | $CH_3$ | $CH_3$ | H | H | |
| 15.22 | $CH_3$ | $CH_3$ | $CH_3$ | $CH_3$ | 1-Cl | H | |
| 15.23 | $-CH_2CH_2-$ | | H | H | H | H | |
| 15.24 | $-CH_2CH_2-$ | | H | H | 1-Cl | H | |
| 15.25 | $-CH_2CH_2-$ | | H | H | 3-Cl | H | |
| 15.26 | $-CH_2CH_2-$ | | H | H | 1-Cl | 3-Cl | |
| 15.27 | H | H | $CH_3$ | H | H | H | |
| 15.28 | $C_2H_5$ | H | H | H | H | H | |

Tabelle 15 (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 15.29 | $C_2H_5$ | $C_2H_5$ | H | H | H | H | |
| 15.30 | $C_2H_5$ | $C_2H_5$ | H | H | 1-Cl | H | |
| 15.31 | $C_2H_5$ | $C_2H_5$ | H | H | 3-Cl | H | |
| 15.32 | $C_2H_5$ | $C_2H_5$ | H | H | 1-Cl | 3-Cl | |
| 15.33 | $C_3H_7$-i | H | H | H | H | H | |
| 15.34 | $-(CH_2)_4-$ | | H | H | H | H | |
| 15.35 | $-(CH_2)_4-$ | | H | H | 1-Cl | 3-Cl | |
| 15.36 | $CH_2CH=CH_2$ | H | H | H | H | H | |
| 15.37 | $CH_2CH=CH_2$ | $CH_3$ | H | H | H | H | |
| 15.38 | $C_4H_9$-n | H | H | H | H | H | |

Tabelle 16:

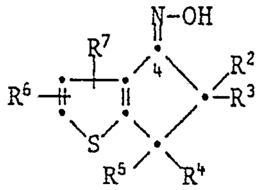

| Verb. Nr. | $R^2$ | $R^3$ | $R^4$ | $R^5$ | $R^6$ | $R^7$ | phys. Daten |
|---|---|---|---|---|---|---|---|
| 16.01 | H | H | H | H | H | H | |
| 16.02 | $CH_3$ | H | H | H | H | H | |
| 16.03 | $CH_3$ | H | $CH_3$ | H | H | H | |
| 16.04 | $CH_3$ | H | $CH_3$ | $CH_3$ | H | H | |
| 16.05 | $CH_3$ | H | H | H | 2-$CH_3$ | H | |
| 16.06 | $CH_3$ | H | H | H | 2-Cl | H | |
| 16.07 | $CH_3$ | H | H | H | 3-$CH_3$ | H | |
| 16.08 | $CH_3$ | H | H | H | 3-F | H | |
| 16.09 | $CH_3$ | H | H | H | 2-$CH_3$ | 3-Br | |
| 16.10 | $CH_3$ | H | H | H | 2-Cl | 3-Cl | |
| 16.11 | $CH_3$ | $CH_3$ | H | H | H | H | |
| 16.12 | $CH_3$ | $CH_3$ | $CH_3$ | H | H | H | |
| 16.13 | $CH_3$ | $CH_3$ | $CH_3$ | $CH_3$ | H | H | |
| 16.14 | $CH_3$ | $CH_3$ | H | H | 2-$CH_3$ | H | |
| 16.15 | $CH_3$ | $CH_3$ | H | H | 2-Cl | H | Smp. 112-114°C |
| 16.16 | $CH_3$ | $CH_3$ | H | H | 2-$C_2H_5$ | H | |
| 16.17 | $CH_3$ | $CH_3$ | H | H | 2-$C_4H_9$-t | H | |
| 16.18 | $CH_3$ | $CH_3$ | H | H | 2-CN | H | |
| 16.19 | $CH_3$ | $CH_3$ | H | H | 2-$NO_2$ | H | |
| 16.20 | $CH_3$ | $CH_3$ | H | H | 2-$CH_3$ | 3-Br | |
| 16.21 | $CH_3$ | $CH_3$ | H | H | 2-$CH_3$ | 3-$CH_3$ | |
| 16.22 | $CH_3$ | $CH_3$ | H | H | 3-$CH_3$ | H | |
| 16.23 | $CH_3$ | $CH_3$ | H | H | 3-F | H | |
| 16.24 | $CH_3$ | $CH_3$ | H | H | 3-$NO_2$ | H | |
| 16.25 | $CH_3$ | $CH_3$ | H | H | 3-Cl | H | |
| 16.26 | $-CH_2-CH_2-$ | | H | H | H | H | |
| 16.27 | $-CH_2CH_2-$ | | H | H | 2-$CH_3$ | H | |
| 16.28 | $-CH_2CH_2-$ | | H | H | 2-Cl | H | |
| 16.29 | H | H | $CH_3$ | H | H | H | |

Tabelle 16: (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 16.30 | H | H | $CH_3$ | $CH_3$ | H | H | |
| 16.31 | $C_2H_5$ | H | H | H | H | H | |
| 16.32 | $C_2H_5$ | $CH_3$ | H | H | H | H | |
| 16.33 | $C_2H_5$ | $C_2H_5$ | H | H | H | H | |
| 16.34 | $C_2H_5$ | $C_2H_5$ | H | H | 2-$CH_3$ | H | |
| 16.35 | $C_2H_5$ | $C_2H_5$ | H | H | 3-$CH_3$ | - | |
| 16.36 | $C_2H_5$ | $C_2H_5$ | H | H | 2-Cl | H | |
| 16.37 | $C_2H_5$ | $C_2H_5$ | H | H | 2-Cl | 3-$CH_3$ | |
| 16.38 | $C_2H_5$ | $C_2H_5$ | $CH_3$ | H | H | H | |
| 16.39 | $C_3H_7$-n | H | H | H | H | H | |
| 16.40 | $C_3H_7$-i | H | H | H | H | H | |
| 16.41 | $-(CH_2)_4-$ | | H | H | H | H | |
| 16.42 | $-(CH_2)_4-$ | | $CH_3$ | H | H | H | |
| 16.43 | $-(CH_2)_4-$ | | H | H | 2-Cl | H | |
| 16.44 | $-(CH_2)_4-$ | | H | H | 2-$CH_3$ | H | |
| 16.45 | $-(CH_2)_4-$ | | H | H | 3-$CH_3$ | H | |
| 16.46 | $-CH_2CH=CH_2$ | H | H | H | H | H | |
| 16.47 | $CH_2CH=CH_2$ | $CH_3$ | H | H | H | H | |
| 16.48 | $C_4H_9$-n | H | H | H | H | H | |

Tabelle 17:

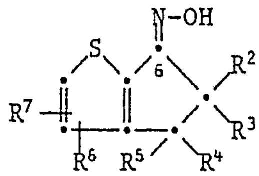

| Verb. Nr. | $R^2$ | $R^3$ | $R^4$ | $R^5$ | $R^6$ | $R^7$ | phys. Daten |
|---|---|---|---|---|---|---|---|
| 17.01 | H | H | H | H | H | H | |
| 17.02 | $CH_3$ | H | H | H | H | H | Smp.126-128°C |
| 17.03 | $CH_3$ | H | $CH_3$ | H | H | H | |
| 17.04 | $CH_3$ | H | $CH_3$ | $CH_3$ | H | H | |
| 17.05 | $CH_3$ | H | H | H | 2-$CH_3$ | H | |
| 17.06 | $CH_3$ | H | H | H | 2-Cl | H | |
| 17.07 | $CH_3$ | H | H | H | 2-$NO_2$ | H | |
| 17.08 | $CH_3$ | H | H | H | 3-Br | 2-$CH_3$ | |
| 17.09 | $CH_3$ | H | H | H | 2-Cl | 3-Cl | |
| 17.10 | $CH_3$ | $CH_3$ | H | H | H | H | |
| 17.11 | $CH_3$ | $CH_3$ | $CH_3$ | H | H | H | |
| 17.12 | $CH_3$ | $CH_3$ | $CH_3$ | $CH_3$ | H | H | |
| 17.13 | $CH_3$ | $CH_3$ | H | H | 2-$CH_3$ | H | |
| 17.14 | $CH_3$ | $CH_3$ | H | H | 2-Cl | H | |
| 17.15 | $CH_3$ | $CH_3$ | H | H | 2-$NO_2$ | H | |
| 17.16 | $CH_3$ | $CH_3$ | H | H | 2-CN | H | |
| 17.17 | $CH_3$ | $CH_3$ | H | H | 2-$C_4H_9$-t | H | |
| 17.18 | $CH_3$ | $CH_3$ | H | H | 3-Br | 2-$CH_3$ | |
| 17.19 | $CH_3$ | $CH_3$ | H | H | 2-Cl | 3-Cl | |
| 17.20 | $CH_3$ | $CH_3$ | H | H | 2-$NO_2$ | 3-$CH_3$ | |
| 17.21 | $CH_3$ | $CH_3$ | H | H | 3-$CH_3$ | H | |
| 17.22 | $CH_3$ | $CH_3$ | H | H | 3-CN | H | |
| 17.23 | $-CH_2CH_2-$ | | H | H | H | H | |
| 17.24 | $-CH_2CH_2-$ | | H | H | 2-$CH_3$ | H | |
| 17.25 | $-CH_2CH_3-$ | | H | H | 2-Cl | H | |
| 17.26 | $-CH_2CH_2-$ | | H | H | 2-$NO_2$ | H | |
| 17.27 | H | H | $CH_3$ | H | H | H | |
| 17.28 | H | H | $CH_3$ | $CH_3$ | H | H | |
| 17.29 | $C_2H_5$ | H | H | H | H | H | |

Tabelle 17 (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 17.30 | $C_2H_5$ | $CH_3$ | H | H | H | H | |
| 17.31 | $C_2H_5$ | $C_2H_5$ | H | H | H | H | |
| 17.32 | $C_2H_5$ | $C_2H_5$ | H | H | 2-$CH_3$ | H | |
| 17.33 | $C_2H_5$ | $C_2H_5$ | H | H | 2-Cl | H | |
| 17.34 | $C_2H_5$ | $C_2H_5$ | H | H | 3-$CH_3$ | 2-Cl | |
| 17.35 | $C_2H_5$ | $C_2H_5$ | $CH_3$ | H | H | H | |
| 17.36 | $C_3H_7$-n | H | H | H | H | H | |
| 17.37 | $C_3H_7$-i | H | H | H | H | H | |
| 17.38 | $-(CH_2)_4-$ | | H | H | H | H | |
| 17.39 | $-(CH_2)_4-$ | | $CH_3$ | H | H | H | |
| 17.40 | $-(CH_2)_4-$ | | H | H | 2-$CH_3$ | H | |
| 17.41 | $-(CH_2)_4-$ | | H | H | 2-Cl | H | |
| 17.42 | $-CH_2CH=CH_2$ | H | H | H | H | H | |
| 17.43 | $CH_2C\equiv CH$ | H | H | H | H | H | |
| 17.44 | $C_4H_9$-n | H | H | H | H | H | |

Tabelle 18:

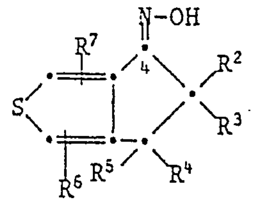

| Verb. Nr. | $R^2$ | $R^3$ | $R^4$ | $R^5$ | $R^6$ | $R^7$ | phys. Daten |
|---|---|---|---|---|---|---|---|
| 18.01 | H | H | H | H | H | H | |
| 18.02 | H | H | H | H | 1-Cl | 3-Cl | |
| 18.03 | H | H | H | H | 3-Cl | H | |
| 18.04 | H | H | H | H | 1-Cl | H | |
| 18.05 | H | H | H | H | $1-CH_3$ | $3-CH_3$ | |
| 18.06 | $CH_3$ | H | H | H | H | H | |
| 18.07 | $CH_3$ | H | H | H | 1-Cl | H | |
| 18.08 | $CH_3$ | H | H | H | 3-Cl | H | |
| 18.09 | $CH_3$ | H | H | H | 1-Cl | 3-Cl | |
| 18.10 | $CH_3$ | H | H | H | $1-CH_3$ | $3-CH_3$ | |
| 18.11 | $CH_3$ | H | $CH_3$ | H | H | H | |
| 18.12 | $CH_3$ | $CH_3$ | H | H | H | H | |
| 18.13 | $CH_3$ | $CH_3$ | H | H | 1-Cl | H | |
| 18.14 | $CH_3$ | $CH_3$ | H | H | 3-Cl | H | |
| 18.15 | $CH_3$ | $CH_3$ | H | H | 1-Cl | 3-Cl | |
| 18.16 | $CH_3$ | $CH_3$ | H | H | $1-CH_3$ | $3-CH_3$ | |
| 18.17 | $CH_3$ | $CH_3$ | H | H | $3-CH_3$ | H | |
| 18.18 | $CH_3$ | $CH_3$ | H | H | $1-NO_2$ | H | |
| 18.19 | $CH_3$ | $CH_3$ | $CH_3$ | H | H | H | |
| 18.20 | $CH_3$ | $CH_3$ | $CH_3$ | H | 1-Cl | H | |
| 18.21 | $CH_3$ | $CH_3$ | $CH_3$ | $CH_3$ | H | H | |
| 18.22 | $CH_3$ | $CH_3$ | $CH_3$ | $CH_3$ | 1-Cl | H | |
| 18.23 | $-CH_2CH_2-$ | | H | H | H | H | |
| 18.24 | $-CH_2CH_2-$ | | H | H | 1-Cl | H | |
| 18.25 | $-CH_2CH_2-$ | | H | H | 3-Cl | H | |
| 18.26 | $-CH_2CH_2-$ | | H | H | 1-Cl | 3-Cl | |
| 18.27 | H | H | $CH_3$ | H | H | H | |
| 18.28 | $C_2H_5$ | H | H | H | H | H | |

Tabelle 18 (Fortsetzung)

| Verb. Nr. | $R^2$ | $R^3$ | $R^4$ | $R^5$ | $R^6$ | $R^7$ | phys. Daten |
|---|---|---|---|---|---|---|---|
| 18.29 | $C_2H_5$ | $C_2H_5$ | H | H | H | H | |
| 18.30 | $C_2H_5$ | $C_2H_5$ | H | H | 1-Cl | H | |
| 18.31 | $C_2H_5$ | $C_2H_5$ | H | H | 3-Cl | H | |
| 18.32 | $C_2H_5$ | $C_2H_5$ | H | H | 1-Cl | 3-Cl | |
| 18.33 | $C_3H_7$-i | H | H | H | H | H | |
| 18.34 | $-(CH_2)_4-$ | | H | H | H | H | |
| 18.35 | $-(CH_2)_4-$ | | H | H | 1-Cl | 3-Cl | |
| 18.36 | $CH_2CH{=}CH_2$ | H | H | H | H | H | |
| 18.37 | $CH_2CH{=}CH_2$ | $CH_3$ | H | H | H | H | |
| 18.38 | $C_4H_9$-n | H | H | H | H | H | |

Formulierungsbeispiele

Beispiel F1: Formulierungsbeispiele für flüssige Wirkstoffe der Formel I (% = Gewichtsprozent)

a) Emulsionskonzentrate

| | a) | b) | c) |
|---|---|---|---|
| Wirkstoff der Formel I | 20 % | 40 % | 50 % |
| Ca-Dodecylbenzol-sulfonat | 5 % | 8 % | 5,8 % |
| Ricinusöl-polyäthylenglykoläther (36 Mol AeO) | 5 % | - | - |
| Tributylphenol-polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

b) Lösungen

| | a) | b) | c) | d) |
|---|---|---|---|---|
| Wirkstoff der Formel I | 80 % | 10 % | 5 % | 95 % |
| Aethylenglykol-mono-methyläther | 20 % | - | - | - |
| Polyäthylenglykol MG 400 | - | 70 % | - | - |
| N-Methyl-2-pyrroli-don | - | 20 % | - | - |
| Epoxidiertes Kokosnussöl | - | - | 1 % | 5 % |
| Benzin (Siedegrenzen 160-190°) | - | - | 94 % | - |

Die Lösungen sind zur Anwendung in form kleinster Tropfen geeignet.

c) Granulate

| | a) | b) |
|---|---|---|
| Wirkstoff der Formel I | 5 % | 10 % |
| Kaolin | 94 % | - |
| Hochdisperse Kieselsäure | 1 % | |
| Attapulgit | - | 90 % |

Der Wirkstoff wird in Methylenchlorid gelöst, auf den Träger aufgesprüht und das Lösungsmittel anschliessend im Vakuum abgedampft.

d) Stäubemittel

| | a) | b) |
|---|---|---|
| Wirkstoff der Formel I | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemdittel.

Beispiel F2: Formulierungsbeispiele für feste Wirkstoffe der Formel I (% = Gewichtsprozent)

a) Spritzpulver

| | a) | b) |
|---|---|---|
| Wirkstoff Nr. 1.14 | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther (7-8 Mol AeO) | - | 2 % |
| Hochdisperse Kieselsäure | 5 % | 27 % |
| Kaolin | 67 % | - |

Der Wirkstoff wird mit den Zusatzstoffen gut vermischt und einer geeigneten Mühle gut vermahlen. Man erhält Spritzpulver, die sich mit Wasser zur Suspension jeder gewünschten Konzentration verdünnen lassen.

b) Emulsions-Konzentrat

| | |
|---|---|
| Wirkstoff Nr. 1.14 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff Nr. 1.14 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff Nr. 2.13 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff Nr. 2.13 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff Nr. 2.13 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Pre-emergente Herbizid-Wirkung

Im Gewächshaus wird unmittelbar nach der Einsaat der Versuchspflanzen in Saatschalen die Erboderfläche mit einer wässrigen Dispersion der Wirkstoffe, erhalten aus einem 25%igen Emulsionskonzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen nach der folgenden Notenskala ausgewertet.

1 : Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

In diesem Versuch zeigen die Verbindungen der Tabellen 1 bis 3 starke Herbizidwirkung.

Testresultate: preemergente Prüfung

Aufwandmange: 4 kg Aktivsubstanz pro Hektar

| Verb. Nr. | Avena | Setaria | Sinapis | Stellaria |
|---|---|---|---|---|
| 1.14 | 3 | 1 | 2 | 2 |
| 2.13 | 4 | 1 | 2 | 1 |

Beispiel B2: Post-emergente Herbizid-Wirkung (Kontaktherbizid)

Eine Anzahl Unkräuter, sowohl monokotyle wir dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage

nach der Behandlung wird der Versuch nach der gleichen Notenskala wie im preemergenten Versuch ausgewertet.

Auch in diesem Versuch zeigen die Verbindungen der Tabellen 1 bis 3 gute Herbizidwirkung.

Testresultate: postemergente Prüfung

Aufwandmenge: 4 kg Aktivsubstanz pro Hektar

| Verb. Nr. | Avena | Setaria | Lolium | Solanum | Sinapis | Stellaria | Phaseolus |
|---|---|---|---|---|---|---|---|
| 1.14 | 5 | 3 | 5 | 2 | 2 | 3 | 2 |
| 2.13 | 6 | 4 | 4 | 3 | 2 | 3 | 2 |

Beispiel B3: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet.

In diesem Versuch zeigen die Verbindungen der Tabellen 1 bis 3 eine sehr starke Herbizidwirkung.

Beispiel B4: Herbizidwirkung für Wasserreis (paddy)

Die Wasserunkräuter Echinochloa crus galli und Monocharia vaginalis werden in Plastikbechern (60 cm$^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die dabei verwendete Dosis entspricht einer Wirkstoffmenge von 4 kg AS pro Hektar (Spritzbrühmenge = 550 l/ha). Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation nach der gleichen Notenskala wie im preemergenten Versuch statt. Die Verbindungen der Tabellen 1 bis 3 schädigen dabei die Unkräuter, nicht aber den Reis.

Testresultate: postemergente Prüfung

Aufwandmenge: 4 kg Aktivsubstanz pro Hektar

| Verb. Nr. | Echinochloa | Monochoria |
|---|---|---|
| 1.14 | 1 | 1 |
| 2.13 | 1 | 1 |

**Patentansprüche**

1. 5,6-Dihydrocyclopentathiophenyl-imidazol-Derivate der Formel I

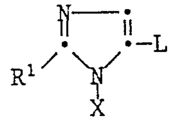

(I),

stereochemisch isomere Formen davon, sowie deren Salze, worin X einen Rest der Formel

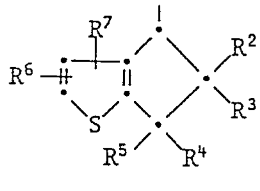

X1

,

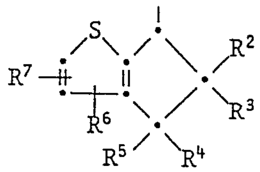

X2

oder

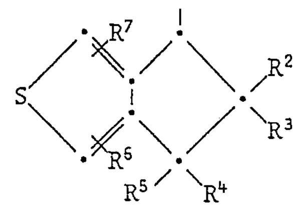

X3

$R^1$ Wasserstoff oder -SH, undL Cyano, -COOH, $-COOR^8$, $-COSR^8$, $-CONR^9R^{10}$, $-CO-R^{11}$, $-CH_2-O-R^{12}$ oder eine Gruppe

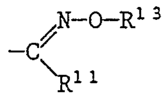

oder

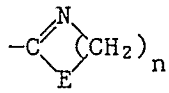

bedeuten, wobei
E für Sauerstoff, Schwefel, -N($C_1$-$C_4$Alkyl)- oder -NH-,
n für die Zahl zwei oder drei,
$R^2$ und $R^3$ unabhängig voneinander für Wasserstoff, $C_1$-$C_4$-Alkyl, $C_2$-$C_4$-Alkenyl oder $C_2$-$C_4$-Alkinyl oder gemeinsam für eine spiro cyclisch verknüpfte $C_2$-$C_6$-Alkylenkette,
$R^4$ und $R^5$ unabhängig voneinander für Wasserstoff oder $C_1$-$C_4$-Alkyl,
$R^6$ für Wasserstoff, Cyano, Halogen, $C_1$-$C_4$-Alkyl, $C_1$-$C_4$-Halogenalkyl, oder Nitro,
$R^7$ für Wasserstoff, Halogen oder $C_1$-$C_4$-Alkyl,
$R^8$ für $C_1$-$C_6$-Alkyl, $C_3$-$C_7$-Cycloalkyl, $C_2$-$C_6$-Alkoxyalkyl, Benzyl, $-CR^{14}R^{15}$-$C_2$-$C_4$-Alkenyl oder $-CR^{14}R^{15}$-$C_2$-$C_4$-Alkinyl,
$R^9$ für Wasserstoff, $C_1$-$C_4$-Alkyl, $C_1$-$C_4$-Alkoxy, Benzyl, $-CR^{14}R^{15}$-$C_2$-$C_4$-Alkenyl oder $-CR^{14}R^{15}$-$C_2$-$C_4$-Alkinyl,
$R^{10}$ für Wasserstoff oder $C_1$-$C_4$-Alkyl, oder
$R^9$ und $R^{10}$ zusammen mit dem sie tragenden Stickstoffatom für einen Pyrrolidin-, Piperidin- oder Morpholinrest,
$R^{11}$ für Wasserstoff, $C_1$-$C_6$-Alkyl, $C_3$-$C_7$-Cycloalkyl, $C_1$-$C_4$-Halogenalkyl oder gegebenenfalls durch $C_1$-$C_4$-Alkyl, $C_1$-$C_4$-Alkoxy, Nitro oder Halogen substituiertes Phenyl,
$R^{12}$ für Wasserstoff, $C_1$-$C_6$-Alkyl, gegebenenfalls durch $C_1$-$C_4$-Alkyl, $C_1$-$C_4$-Alkoxy, Halogen oder Nitro substituiertes Phenyl, $C_1$-$C_6$-Alkylcarbonyl, $C_1$-$C_4$-Halogenalkylcarbonyl oder gegebenenfalls durch $C_1$-$C_4$-Alkyl, $C_1$-$C_4$-Alkoxy, Halogen oder Nitro substituiertes Benzoyl,
$R^{13}$ für $C_1$-$C_4$-Alkyl, $-CR^{14}R^{15}$-$C_2$-$C_4$-Alkenyl, Benzyl oder $-CR^{14}R^{15}$-$C_2$-$C_4$-Chloralkenyl,
und
$R^{14}$ und $R^{15}$ unabhängig voneinander für Wasserstoff oder $C_1$-$C_4$-Alkyl stehen.

2. Verbindungen gemäss Anspruch 1, dadurch gekennzeichnet, dass L für $C_1$-$C_4$-Alkoxycarbonyl steht.

3. Verbindungen gemäss Anspruch 1, dadurch gekennzeichnet, dass X für den Rest

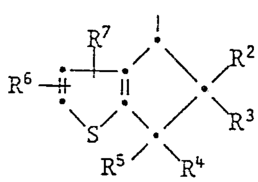

X1

steht, worin
$R^1$ und $R^3$ unabhängig voneinander Wasserstoff oder $C_1$-$C_4$-Alkyl, $R^4$ und $R^5$ Wasserstoff und $R^6$ Wasserstoff, $C_1$-$C_4$-Alkyl oder Halogen bedeuten.

4. Verbindungen gemäss Anspruch 1, dadurch gekennzeichnet, dass X für den Rest

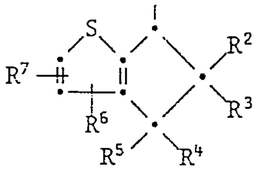

steht, worin
$R^2$ und $R^3$ unabhängig voneinander Wasserstoff oder $C_1$-$C_4$-Alkyl, $R^4$ und $R^5$ Wasserstoff und $R^6$

Wasserstoff, $C_1$-$C_4$-Alkyl oder Halogen bedeuten.

5. 1-(5,6-Dihydro-5,5-dimethyl-4H-cyclopenta[b]thiophen-4-yl)-5-imidazolcarbonsäure-methylester oder 1-(5,6-Dihydro-5,5-dimethyl-4H-cyclopenta[b]thiophen-6-yl)-5-imidazolcarbonsäure-methylester gemäss Anspruch 1.

6. Verfahren zur Herstellung der Verbindungen der Formel, worin $R^1$ für Wasserstoff steht, dadurch gekennzeichnet, dass man ein Amin der Formel II

$X$-$NH_2$ (II)

worin X die unter Formel I im Anspruch 1 gegebene Bedeutung hat, mit einem N-Cyan-Imidoameisensäureester der Formel III

R-O-CH = N-CN (III)

worin R für $C_1$-$C_4$-Alkyl steht, kondensiert, das entstehende N-Cyanoformamidin der formel IV

X-NH-CH = N-CN (IV)

worin X die unter Formel I im Anspruch 1 gegebene Bedeutung hat, mit einem Halogenessigsäureester der Formel V

Hal-$CH_2$-L (V)

worin Hal für Chlor oder Brom und L für $C_1$-$C_6$-Alkoxycarbonyl stehen, in Gegenwart einer Base alkyliert, das entstehende Zwischenprodukt der Formel VI

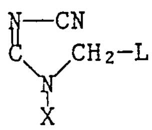

(VI)

worin X die unter Formel I im Anspruch 1 gegebene Bedeutung hat und L für $C_1$-$C_6$-Alkoxycarbonyl steht, in Gegenwart einer Base zum 4-Amino-imidazol der Formel VII

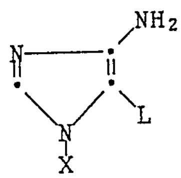

(VII)

worin X die unter Formel I im Anspruch 1 gegebene Bedeutung hat und L für $C_1$-$C_6$-Alkoxycarbonyl steht, cyclisiert und diese Verbindung unter Diazotierung und Stickstoffabspaltung in das Produkt der Formel I, worin L für $C_1$-$C_6$-Alkoxycarbonyl und $R^1$ für Wasserstoff stehen, überführt und gewünschtenfalls die Gruppe L in 5-Stellung gemäss den übrigen Bedeutungen der Definition unter Formel I im Anspruch 1 derivatisiert.

7. Verfahren zur Herstellung der Verbindungen der Formel I, dadurch gekennzeichnet, dass man ein Amin der Formel II

X-$NH_2$ (II)

worin X die unter Formel I im Anspruch 1 gegebene Bedeutung hat, mit einem Halogenessigsäureester der Formel V

Hal-$CH_2$-L (V)

worin Hal für Chlor oder Brom und L für $C_1$-$C_6$-Alkoxycarbonyl stehen, in Gegenwart einer Base alkyliert, den entstehenden Glycinester der Formel VIII

X-NH-$CH_2$-L (VIII)

worin X die unter Formel I im Anspruch 1 gegebene Bedeutung hat und L für $C_1$-$C_6$-Alkoxycarbonyl steht, in Gegenwart von Essigsäureanhydrid mit Ameisensäure formyliert, das entstehende Zwischenprodukt der Formel IX

HCO-$\underset{X}{N}$-$CH_2$-L (IX)

worin X die unter Formel I im Anspruch 1 gegebene Bedeutung hat und L für $C_1$-$C_6$-Alkoxycarbonyl steht, in Gegenwart einer Alkalibase mit einem Ameisensäure-$C_1$-$C_4$-alkylester umsetzt, das entstehende Zwischenprodukt der Formel X

HCO-$\underset{X}{N}$ – $\underset{L}{C}$ =CH-O-M (X)

worin X die unter Formel I gegebene Bedeutung hat und L für $C_1$-$C_6$-Alkoxycarbonyl und M für ein Alkalikation steht, mit Thiocyansäure zum Produkt der Formel I, worin $R^1$ für -SH und L für $C_1$-$C_6$-Alkoxycarbonyl stehen, cyclisiert und diese Verbindung gewünschtenfalls in der 2-Stellung desulfuriert und gewünschtenfalls die Gruppe L in 5-Stellung gemäss den übrigen Bedeutungen der Definition unter Formel I im Anspruch 1 derivatisiert.

8. Herbizides Mittel, dadurch gekennzeichnet, dass es einer wirksamen Menge einer Verbindung der Formel I einen oder mehrere Träger- und/oder Zuschlagstoffe enthält.

9. Verfahren zur Bekämpfung von unerwünschtem Pflanzenwuchs, dadurch gekennzeichnet, dass man

die Pflanzen oder deren Standort mit einer herbizid wirksamen Menge eines Wirkstoffs der Formel I behandelt.

10. Amine der Formel

$H_2N$-X (II)

worin X die unter Formel I im Anspruch 1 gegebene Bedeutung hat, unter Ausnahme der Verbindung 4-Amino-5,6-dihydro-4H-cyclopenta[b]thiophen.

11. 4-Amino-imidazole der Formel VII

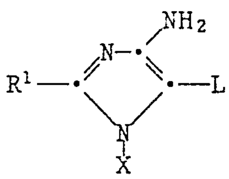

(VII)

worin $R^1$, X und L die unter Formel I im Anspruch 1 gegebene Bedeutungen haben.

12. Oxime der Formel XI

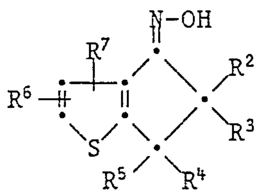

(XI)

worin $R^2$, $R^3$, $R^4$, $R^5$, $R^6$ und $R^7$ die unter Formel I im Anspruch 1 gegebenen Bedeutungen haben, mit Ausnahme der Verbindungen 5,6-Dihydro5-methyl-4H-cyclopenta[b]thiophen-4-on-oxim und 5,6-Dihydro-2,3-dimethyl-4H-cyclopenta[b]thiophen-4-on oxim der Oxime der Formel XII

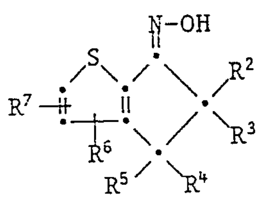

(XII)

worin $R^2$, $R^3$, $R^4$, $R^5$, $R^6$ und $R^7$ die unter Formel I im Anspruch 1 gegebenen Bedeutungen haben, mit Ausnahme der Verbindungen 5,6-Dihydro-5-methyl-6H-cyclopenta[b]thiophen-6-on-oxim, 5,6-Dihydro-6H-cyclopenta[b]thiophen-6-on-oxim und 2-Chlor-5,6-dihydro-5-methyl-6H-cyclopenta[b]thiophen-6-on-oxim oder Oxime der Formel XIII

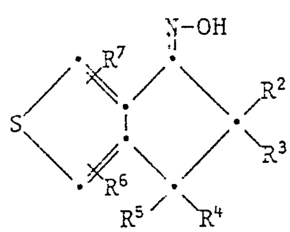

(XIII)

worin $R^2$, $R^3$, $R^4$, $R^5$, $R^6$ und $R^7$ die unter Formel I im Anspruch 1 gegebenen Bedeutungen haben, mit Ausnahme der Verbindungen 5,6-Dihydro-1,3-dimethyl-4H-cyclopenta[c]thiophen-4-on-oxim, 1-Chlor-5,6-dihydro-4H-cyclopenta[c]thiophen-4-on oxim, 5,6-Dihydro-1,3,5-trimethyl-4H-cyclopenta[c]thiophen-4-on-oxim und 1,3-Dichlor-5,6-dihydro-4H-cyclopenta[c]thiophen-4-on-oxim.

13. 5,6-Dihydro-4H-cyclopenta[b]thiophen-4-one der Formel

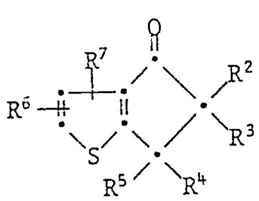

(XIV)

worin $R^2$, $R^3$, $R^4$, $R^5$, $R^6$ und $R^7$ die unter Formel I im Anspruch 1 gegebenen Bedeutungen haben, mit der Massgabe, dass mindestens zwei der Reste $R^2$, $R^3$, $R^4$ und $R^5$ eine von Wasserstoff verschiedene Bedeutung haben oder 5,6-Dihydro-6H-cyclopenta[b]thiophen-6-one der Formel

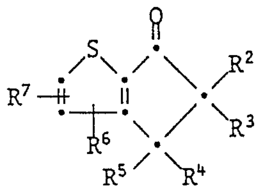

(XV)

worin $R^2$, $R^3$, $R^4$, $R^5$, $R^6$ und $R^7$ die unter Formel I im Anspruch 1 gegebenen Bedeutungen haben, mit der Massgabe, dass mindestens zwei der Reste $R^2$, $R^3$, $R^4$ und $R^5$ eine von Wasserstoff verschiedene Bedeutung haben, mit Ausnahme der Verbindung 5,6-Dihydro-4,4-dimethyl-4H-cyclopenta[b]thiophen-6-on oder 5,6-Dihydro-4H-cyclopenta[c]thiophen-4-one der Formel

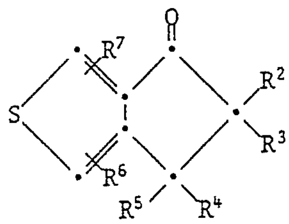

(XVI)

worin $R^2$, $R^3$, $R^4$, $R^5$, $R^6$ und $R^7$ die unter Formel I im Anspruch 1 gegebenen Bedeutungen haben, mit der Massgabe, dass mindestens zwei der Reste $R^2$, $R^3$, $R^4$ und $R^5$ eine von Wasserstoff verschiedene Bedeutung haben.

Europäisches Patentamt

# EUROPÄISCHER RECHERCHENBERICHT

Nummer der Anmeldung

EP 89 81 0423

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 031 266 (SANOFI)<br>* Seite 3; Figuren III,IV *<br>--- | 12 | C 07 D 409/04<br>A 01 N 43/50<br>C 07 D 333/78<br>A 01 N 43/12 |
| A | FR-A-2 584 071 (LIPHA, LYONNAISE INDUSTRIELLE PHARMACEUTIQUE)<br>--- | | |
| D,A | JOURNAL OF PHARMACEUTICAL SCIENCES, Band 52, Nr. 9, 1. September 1963, Seiten 898-901, Washington, US; J. SAM et al.: "Thiaindanones. Thiophene isosteres of indanone"<br>* Insgesamt *<br>--- | | |
| D,A | EP-A-0 207 563 (JANSSEN PHARMACEUTICA N.V.)<br>--- | | |
| D,A | EP-A-0 234 656 (JANSSEN PHARMACEUTICA N.V.)<br>--- | | |
| P,A | EP-A-0 289 066 (JANSSEN PHARMACEUTICA N.V.)<br>----- | | **RECHERCHIERTE SACHGEBIETE (Int. Cl.4)**<br>C 07 D 409/00<br>A 01 N 43/00<br>C 07 D 333/00 |

Der vorliegende Recherchenbericht wurde für alle Patentansprüche erstellt

| Recherchenort | Abschlußdatum der Recherche | Prüfer |
|---|---|---|
| DEN HAAG | 14-09-1989 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0401)